(19) Europäisches Patentamt
European Patent Office
Office européen des brevets

(11) EP 0 800 512 B1

(12) **EUROPEAN PATENT SPECIFICATION**

(45) Date of publication and mention of the grant of the patent:
**05.01.2000 Bulletin 2000/01**

(21) Application number: **95935318.6**

(22) Date of filing: **31.10.1995**

(51) Int Cl.[7]: **C07C 275/16**, A61K 31/17, A61K 7/40, C07C 271/22

(86) International application number:
**PCT/CA95/00626**

(87) International publication number:
**WO 96/20171 (04.07.1996 Gazette 1996/30)**

(54) **ANTIHERPES PEPTIDOMIMETIC COMPOUNDS**

ANTIHERPETISCHE PEPTIDOMIMETISCHE VERBINDUNGEN

COMPOSES PEPTIDOMIMETIQUES ANTI-HERPES

(84) Designated Contracting States:
**AT BE CH DE DK ES FR GB GR IE IT LI LU MC NL PT SE**

(30) Priority: **28.12.1994 CA 2139169**

(43) Date of publication of application:
**15.10.1997 Bulletin 1997/42**

(73) Proprietor: **BOEHRINGER INGELHEIM (CANADA) LTD.**
**Laval, Quebec, H7S 2G5 (CA)**

(72) Inventors:
- **DEZIEL, Robert**
  **Mont-Royal, Quebec H3R 1W9 (CA)**
- **BRUNET, Montse, Llinas**
  **Pierrefonds, Quebec H8Y 2K7 (CA)**
- **MOSS, Neil**
  **Ridgefield, CT 06877 (US)**
- **PLANTE, Raymond**
  **Laval, Quebec H7L 4W8 (CA)**

(74) Representative: **Laudien, Dieter, Dr.**
**Boehringer Ingelheim GmbH**
**Abteilung Patente,**
**Postfach 200**
**55216 Ingelheim (DE)**

(56) References cited:
**EP-A- 0 560 267** **WO-A-94/25046**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 800 512 B1

## Description

This invention relates to peptidomimetic compounds having antiviral properties and to means for using the compounds to treat viral infections. More specifically, the invention relates to peptidomimetic compounds exhibiting activity against herpes viruses, to pharmaceutical compositions comprising the compounds, and to methods of using the compounds to inhibit the replication of herpes virus and to treat herpes infections.

Background of the Invention

Herpes viruses inflict a wide range of diseases against humans and animals. For instance, herpes simplex viruses, types 1 and 2 (HSV-1 and HSV-2), are responsible for cold sores and genital lesions, respectively; varicella zoster virus (VZV) causes chicken pox and shingles; and the Epstein-Barr virus (EBV) causes infectious mononucleosis.

Over the past two decades, a class of compounds known as the purine and pyrimidine nucleoside analogs has received the most attention by investigators in the search for new therapeutic agents for treatment of herpes virus infections. As a result, several nucleoside analogs have been developed as antiviral agents. The most successful to date is acyclovir which is the agent of choice for treating genital herpes simplex infections.

Nevertheless, in spite of some significant advances, the need for effective, safe therapeutic agents for treating herpes viral infections continues to exist. For a review of current therapeutic agents in this area, see R.E. Boehme et al., Annual Reports in Medicinal Chemistry, 29, 145 (1994).

The present application discloses a group of compounds having activity against herpes simplex viruses. The selective action of these compounds against herpes viruses, combined with a wide margin of safety, renders the compounds as desirable agents for combating herpes infections.

The following references disclose peptides or peptidomimetic compounds which have been associated with antiherpes activity:

J.H. Subak-Sharpe et al., UK patent application 2185024, published July 8, 1987,
P. Gaudreau et al., J. Biol. Chem., 262, 12413 (1987),
E.A. Cohen et al., US patent 4,795,740, January 3, 1989,
R. Freidinger et al., US patent 4,814,432, March 21, 1989,
V.M. Garskey et al., US patent 4,837,304, June 6, 1989,
R. Colonno et al., US patent 4,845,195, July 4, 1989,
P. Gaudreau et al., J. Med. Chem., 33, 723 (1990),
J. Adams et al., European patent application 411,334, published February 6, 1991,
R.L. Tolman et al., European patent application 412, 595, published February 13, 1991,
W.T. Ashton et al., European patent application 438,873, published July 31, 1991,
P.L. Beaulieu et al., European patent application 461,546, published December 18, 1991,
P. Gaudreau et al., J. Med. Chem., 35, 346 (1992).
R. Déziel and Y. Guindon, Canadian patent application 2,033,448, published July 1, 1992,
L.L. Chang et al., Bioorganic & Medicinal Chemistry Letters, 2, 1207 (1992),
P.L. Beaulieu et al., European patent application 560 267, published September 15, 1993,
N. Moss et al., J. Med. Chem., 36, 3005 (1993) and
R. Déziel and N. Moss, European patent application 618 226, published October 5, 1994.

The subject peptides of the previous reports can be distinguished from the peptides of the present application by characteristic structural and biological differences.

EP-A-560 267 discloses peptide derivatives which are useful for treating herpes infections. The peptide derivatives are represented by formula

$$A\text{-}B\text{-}D\text{-}CH_2CH\{CH_2C(O)R^1\}C(O)\text{-}NHCH\{CR^2(R^3)COOH\}C(O)\text{-}E$$

where A is an optionally substituted phenylalkanoyl or (phenylalkyl)aminocarbonyl; B is an N-methyl amino acid residue; or A and B together form a saturated or unsaturated alkylaminocarbonyl, or a cycloalkylaminocarbonyl optionally substituent at position 1 of the cycloalkyl; D is an amino acid residue; $R^1$ is alkyl, cycloalkyl, a monosubstituted or a disubstituted amino, or a pyrrolidino, piperidino, morpholino or 4-methylpiperazino; $R^2$ is hydrogen or alkyl; $R^3$ is alkyl; or $R^2$ and $R^3$ together form a cycloalkyl; and E is an alkylamino, an optinally mono- or disubstituted cycloalkylamino wherein the substituent is alkyl or (cycloalkyl)alkyl, a mono- or dialkylhydrazino or E is a monosubstituted alkyl or

(cycloalkyl)alkyl wherein the monosubstituent on the alkyl or on the alkyl portion of the (cycloalkyl)alkyl is $CH_2OH$, C(O)OH, $C(O)NH_2$ or C(O)-O-alkyl. These peptide derivatives can be distinguished from the compounds of the present application by the lack of a 1α-amino-2α,6α-dialkylcyclohexane radical at the N-terminus of the peptide derivatives.

WO-A-94 025 046 is directed to a previously undisclosed use of peptide derivatives, alone or in combination with known antiviral nucleoside analogs, for treating acyclovir-resistant herpes infection. The peptide derivatives are included in the group of more preferred compounds noted in above-mentioned document EP-A-520 267 on page 3, line 37 to page 4, line 5.

Abbreviations and symbols used hereinafter are defined in "Details of the Invention" section of this application.

## Summary of the Invention

The compounds of this invention are represented by formula 1

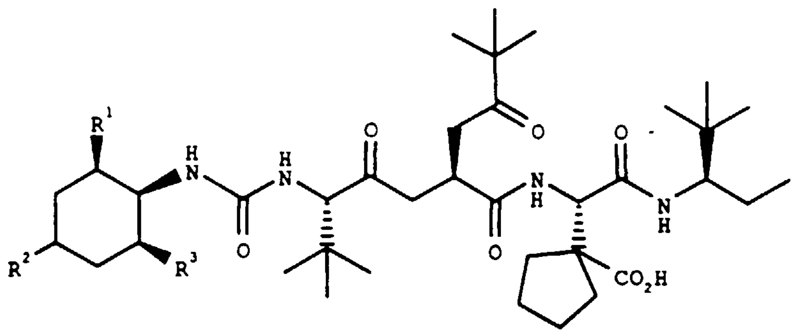

(1)

wherein $R^1$ is (1-3C)alkyl, $R^2$ is hydrogen or (1-3C)alkyl and $R^3$ is (1-3C)alkyl, or a therapeutically acceptable salt thereof.

A preferred group of the compounds of this invention is represented by formula 1 wherein $R^1$ and $R^3$ are both methyl or both ethyl, and $R^2$ is hydrogen, methyl or ethyl, or a therapeutically acceptable salt thereof.

A more preferred group of compounds are represented by formula 1 wherein $R^1$ and $R^3$ are both methyl and $R^2$ is hydrogen or a cis-methyl relative to $R^1$ and $R^3$; or $R^1$ and $R^3$ are both ethyl and $R^2$ is hydrogen; or a therapeutically acceptable salt thereof.

Included within the scope of this invention is a pharmaceutical composition comprising an antiherpes virally effective amount of a compound of formula 1, or a therapeutically acceptable salt thereof, and a pharmaceutically acceptable carrier.

Also included within the scope of this invention is a cosmetic composition comprising a compound of formula 1, or a therapeutically acceptable salt thereof, and a physiologically acceptable carrier suitable for topical application.

Processes for preparing the compounds of formula 1 are described hereinafter.

## Description of the Drawings

Figures 1 and 2 are graphic representations of results obtained from studies involving combinations of acyclovir and a peptidomimetic compound of formula 1. The studies involve the application of the isobole method, described in example 12, to demonstrate the synergistic activity of the combinations against herpes simplex viruses, types 1 and 2, respectively.

## Details of the Invention

### General

Alternatively, formula 1 can be illustrated as:

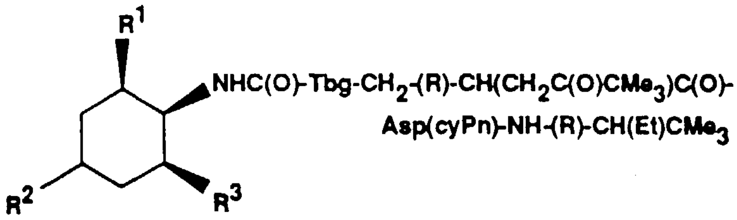

wherein Tbg represents the amino acid residue of (S)-2-amino-3,3-dimethylbutanoic acid, Me and Et represent the alkyl radicals methyl and ethyl, respectively, and Asp(cyPn) represents the amino acid residue of (S)-α-amino-1-carboxycyclopentaneacetic acid.

The term "residue" with reference to an amino acid or amino acid derivative means a radical derived from the corresponding α-amino acid by eliminating the hydroxyl of the carboxy group and one hydrogen of the α-amino group.

The term "(1-3C)alkyl" as used herein means an alkyl radical selected from the group consisting of methyl, ethyl, propyl or isopropyl.

The term "pharmaceutically acceptable carrier" as used herein means a non-toxic, generally inert vehicle for the active ingredient which does not adversely affect the ingredient.

The term "physiologically acceptable carrier" as used herein means an acceptable cosmetic vehicle of one or more non-toxic excipients which do not react with or reduce the effectiveness of the active ingredient contained therein.

The term "effective amount" means a predetermined antiviral amount of the antiviral agent, i.e. an amount of the agent sufficient to be effective against herpes virus *in vivo.*

Process for Preparing the Compounds of Formula 1

In general, the compounds of formula 1 are prepared by known methods using reaction conditions which are known to be suitable for the reactants. Description of the methods are found in standard textbooks such as "Annual Reports In Organic Synthesis - 1994", P.M. Weintraub et al., Eds, Academic Press, Inc., San Diego, CA, USA, 1994 (and the preceding annual reports), "Vogel's Textbook Of Practical Organic Chemistry", B.S. Furniss et al., Eds, Longman Group Limited, Essex, UK, 1986, and "Comprehensive Organic Synthesis", B.M. Trost and I. Fleming, Eds, Pergaman Press, Oxford, UK, 1991, Volumes 1 to 8.

An exception to the latter statement, however, is the unique stereospecific synthesis of a key intermediate for the preparation of the compounds of formula 1. This key intermediate is represented by formula 2

$$W^1\text{-Tbg-}CH_2\text{-(R)-}CH(CH_2C(O)CMe_3)C(O)OW^2 \qquad (2)$$

wherein $W^1$ is an amino protective group, and $W^2$ is a carboxyl protective group. In this instance, $W^2$ is a protective group which can be selectively removed in the presence of the protective group $W^1$. Preferably, $W^1$ is tert-butyloxycarbonyl (Boc) or 2,2,2-trichloroethoxycarbonyl and $W^2$ is benzyl, (4-nitrophenyl)methyl, methyl or ethyl.

The intermediate of formula 2 can be prepared by a stereospecific process illustrated in the following Scheme 1.

Scheme 1

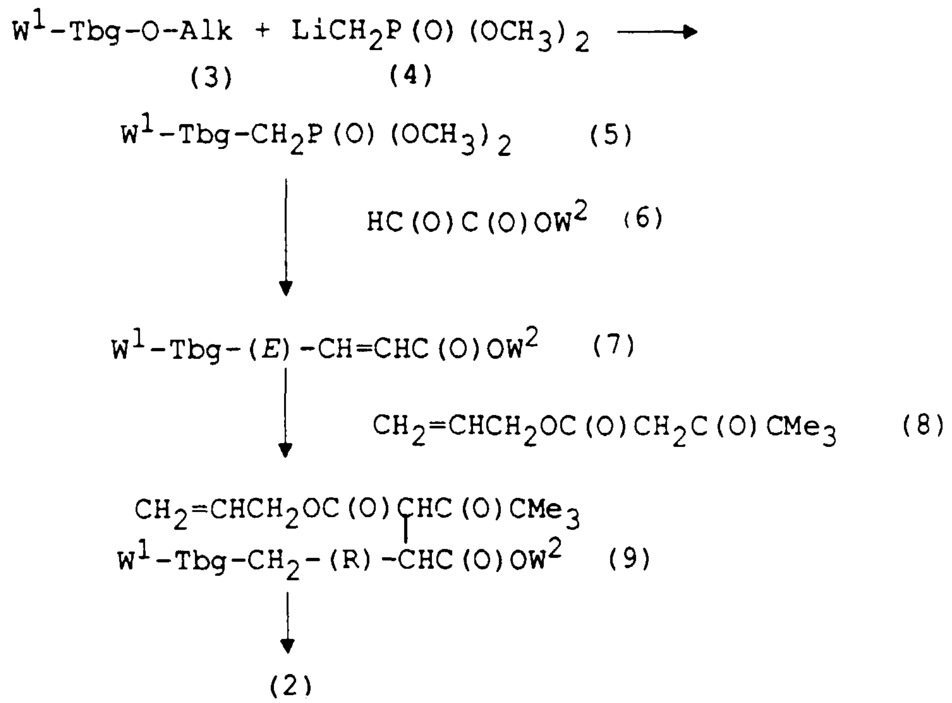


wherein $W^1$ and $W^2$ are as defined herein and Alk is methyl or ethyl.

With reference to the preceding schematic representation, a starting material of formula $W^1$-Tbg-O-Alk (3) is reacted with the reagent $LiCH_2P(O)(OCH_3)_2$ (4) (prepared from $CH_3P(O)(OCH_3)_2$ and butyllithium) to give a phosphonate of formula $W^1$-Tbg-$CH_2P(O)(OCH_3)_2$ (5) . Reaction of the latter phosphonate with a glyoxylyl ester of formula $HC(O)C(O)OW^2$ (6) in the presence a suitable tertiary amine, preferably triethylamine or diisopropylethylamine, affords a γ-keto-α,β-unsaturated ester of formula $W^1$-Tbg-(*E*)-CH=CHC(O)$OW^2$ (7). Reaction of the latter compound with the sodium enolate of a β-ketoester of formula $CH_2$=$CHCH_2OC(O)CH_2C(O)CMe_3$ (8) affords a Michael adduct of formula $W^1$-Tbg-$CH_2$-(R)-CH{CH(C(O)$CMe_3$)-(C(O)$OCH_2CH$=$CH_2$)}C(O)$OW^2$ (9).

Note (1): The β-ketoester of formula 8, i.e. $CH_2$=$CHCH_2OC(O)CH_2C(O)CMe_3$, is prepared readily by reacting the lithium enolate of allyl acetate with trimethylacetyl chloride.

Note (2) : The sodium enolate of the β-ketoester of formula 8 is generated *in* situ from the β-ketoester in the presence of a catalytically effective amount of sodium hydride.

Thereafter, reaction of the Michael adduct of formula 9 with tetrakistriphenylphosphine palladium (0) in the presence of a suitable secondary amine, preferably pyrrolidine or piperidine, similar to the method of R. Déziel, Tetrahedron Letters, 28, 4371 (1987), effects deallylation and subsequent decarboxylation of the allyl ester to give the key intermediate of formula 2.

Noteworthy is the unexpected high stereoselectivity obtained in the Michael addition reaction of the γ-keto-α,β-unsaturated ester of formula 7 with the sodium enolate of the β-ketoester of formula 8 to give the Michael adduct of formula 9. The stereoselectivity of the Michael addition reaction is inferred by the fact that the intermediate of formula 2, derived directly from the Michael adduct, is obtained essentially as a single isomer. The diastereoisomeric purity of the intermediate of formula 2 can be demonstrated by nuclear magnetic resonance studies. The enantiomeric purity of the intermediate of formula 2 can be assessed by removing the amino protective group ($W^1$) and applying the method of J. A. Dale et al., J. Org. Chem., 34, 2543 (1969) to the resulting free amino derivative (see example 4 for more detail).

Thereafter again, the carboxyl protective group ($W^2$) of the key intermediate of formula 2 is selectively removed by standard methods, for example, by hydrogenolysis in the instance wherein $W^2$ is benzyl, to give the corresponding free carboxylic acid derivative (see formula 14 in Scheme 2 below) for incorporation into the process for preparing the compounds of formula 1.

In general, the incorporation of the preceding free carboxylic acid derivative into a process for the preparation of the compounds of formula 1 can be envisaged as a sequence of chemical events wherein a carboxylic acid derivative (representing a first unit) is joined to two other units by first forming an amide bond, and secondly by forming a ureido

bond.

In the following more detailed description of a convenient and practical process for preparing the compounds of formula 1, a certain order of the chemical events is followed. However, it will be appreciated that changes in the order of chemical events are not critical and therefore such changes are deemed to be within the scope of the present invention.

Likewise, it should be appreciated that the intermediate of formula 2 wherein protective group $W^1$ can be selectively removed in the presence of protective group $W^2$, allowing for a change in the order of the chemical events, also is deemed to be within the scope of the present invention. Accordingly, an important aspect of this invention includes a key intermediate of formula 2 in which $W^1$ is an amino protective group for the amine at the N-terminus and $W^2$ is a carboxyl protective group for the carboxyl at the C-terminus of the intermediate, with the proviso that the amino protective group $W^1$ can be selectively removed in the presence of the carboxyl protective group $W^2$ when the terminal amine is destined for the reaction to follow, or that, on the other hand, the carboxyl protective group $W^2$ can be selectively removed in the presence of the amino protective group $W^1$ when the terminal carboxyl is destined for the reaction to follow.

Examples of the intermediates of formula 2 wherein the amino protective group $W^1$ can be selectively removed in the presence of the carboxyl protective group $W^2$ include those in which $W^1$ is tert-butyloxycarbonyl and $W^2$ is benzyl, 2,2,2-trichloroethyl, methyl or ethyl.

More particularly, with respect to an overall process, the compounds of formula 1 can be prepared by a convenient and practical process illustrated in the following Scheme 2.

Scheme 2

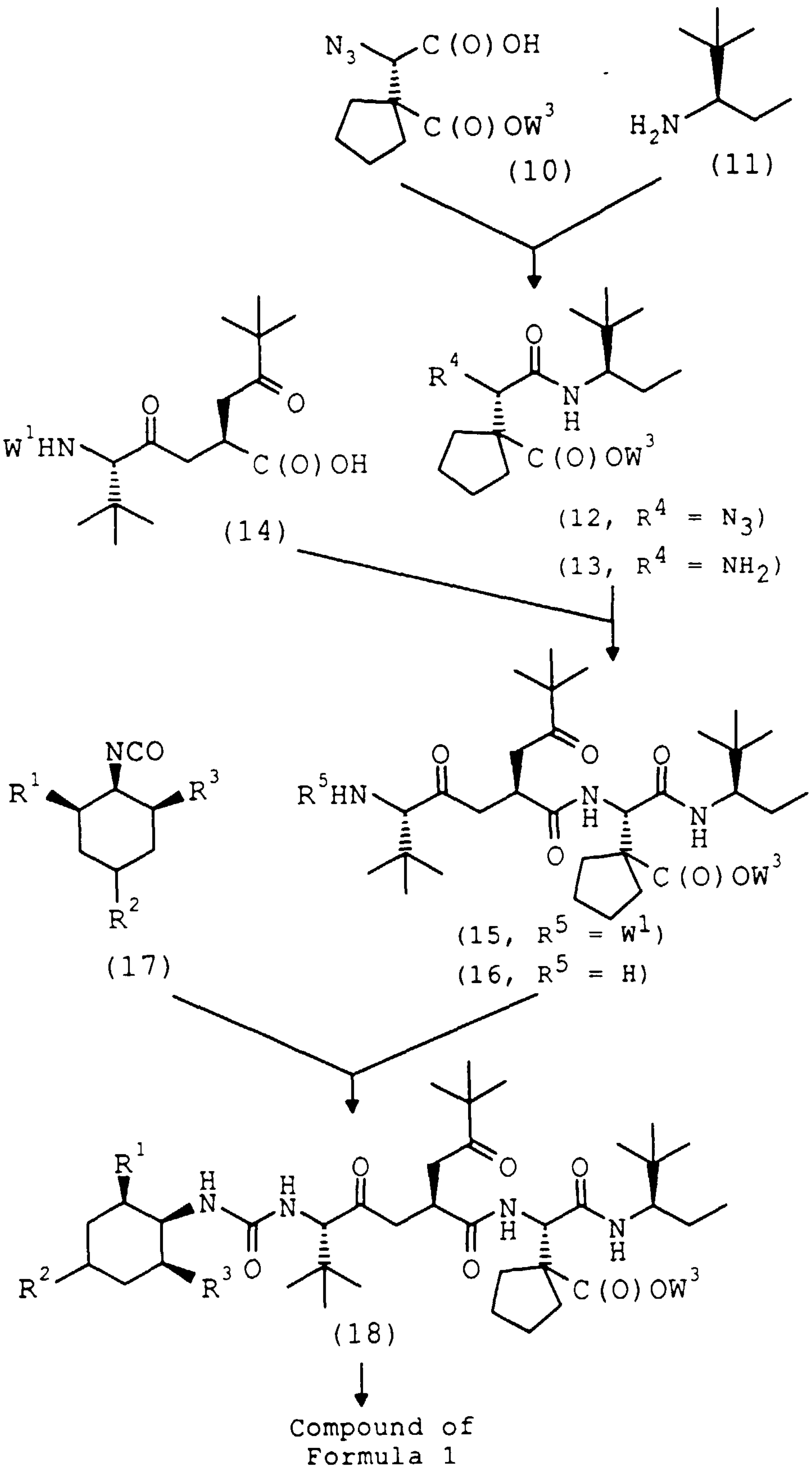

(10)

(11)

(14)

(12, $R^4 = N_3$)

(13, $R^4 = NH_2$)

(17)

(15, $R^5 = W^1$)

(16, $R^5 = H$)

(18)

Compound of Formula 1

In Scheme 2, $W^3$ is a carboxyl protective group (preferably benzyl, tert-butyl or 2,2,2-trichloroethyl), $R^4$ is azido for formula 12 and an amino for formula 13, $R^5$ is $W^1$ as defined herein for formula 15 and a hydrogen for formula 16, and $R^1$, $R^2$ and $R^3$ are as defined herein.

Referring to Scheme 2, a process for preparing compound of formula 1 comprises:

(a) coupling a carboxylic acid derivative of formula 10 with an amine of formula 11 to obtain an α-azidoamide of formula 12,
(b) reducing the α-azidoamide of formula 12 to obtain a corresponding α-aminoamide of formula 13,
(c) coupling the α-amidoamide of formula 13 with a carboxylic acid derivative of formula 14 to obtain a diprotected intermediate of formula 15,
(d) selectively deprotecting the diprotected intermediate of formula 15 to obtain the free N-terminal derivative of formula 16,
(e) reacting the free N-terminal derivative of formula 16 with an isocyanatocyclohexane derivative of formula 17 to obtain a ureido derivative of formula 18, and
(f) deprotecting the latter ureido derivative of formula 18 to obtain the corresponding compound of formula 1, and
(g) if desired transforming the compound of formula 1 into a therapeutically acceptable salt thereof.

The coupling steps (a) and (c) and the deprotecting steps (d) and (f) can be achieved by methods commonly used in peptide synthesis.

More explicitly, the coupling step involves the dehydrative coupling of a free carboxyl of one reactant with the free amino group of the other reactant in the presence of coupling agent to form a linking amide bond. Description of such coupling agents are found in general textbooks on peptide chemistry, for example, M. Bodanszky, "Peptide Chemistry", 2nd rev ed, Springer-Verlag, Berlin, Germany, 1993. Examples of suitable coupling agents are N,N'-dicyclohexylcarbodiimide, 1-hydroxybenzotriazole in the presence of N,N'-dicyclohexylcarbodiimide or N-ethyl-N'-[(3-dimethylamino)propyl]carbodiimide. A very practical and useful coupling agent is the commercially available (benzotriazol-1-yloxy)tri(dimethylamino)phosphonium hexafluorophosphate, either by itself or in the presence of 1-hydroxybenzotriazole. Still another very practical and useful coupling agent is commercially available 2-(1H-benzotriazol-1-yl)-N, N, N', N'-tetramethyluronium tetrafluoroborate.

The coupling reaction is conducted in an inert solvent, e.g. dichloromethane or acetonitrile. An excess of a tertiary amine, e.g. diisopropylethylamine or N-methylmorpholine, is added to maintain the reaction mixture at a pH of about eight. The reaction temperature usually ranges between 0° and 50 °C and the reaction time usually ranges between 15 minutes and 24 hours.

In step (b), the azide group of the α-azidoamide of formula 12 is transformed into a corresponding amine of the α-aminoamide of formula 13 by a reducing agent capable of selectively reducing an azide to an amino group in the presence of an amido group and an ester group. This step can be accomplished conveniently and efficiently by the method of N. Maiti et al., Tetrahedron Letters, 27, 1423 (1986) using stannous chloride as the reducing agent and methanol as the reaction solvent.

In step (e), the free N-terminal derivative of formula 16 is reacted directly with a isocyanatocyclohexane derivative of formula 17 to give the ureido derivative of formula 18. This step is based on the classical method for preparing a urea whereby an isocyanato derivative of the residue to be incorporated is reacted with the terminal amino group of an appropriate fragment. {For examples of this method, see P. Majer and R.S. Randad, J. Org. Chem., 59, 1937 (1994).} The reaction proceeds readily in the presence of an excess of a suitable tertiary amine, for example N-methylmorpholine or diisopropylethylamine. The reaction is conducted in an inert solvent, such as dichloromethane or toluene, and at temperatures usually ranging from -20 °C to 20 °C.

Furthermore, if desired, the compound of formula 1 can be obtained in the form of a therapeutically acceptable salt. Such salts can be considered as biological equivalents of the compounds of formula 1. Examples of such salts (of the carboxy group) are those formed by known methods with the sodium, potassium or calcium cation.

Antiherpes Activity

The antiviral activity of the compounds of formula 1 can be demonstrated by biochemical, microbiological and biological procedures showing the inhibitory effect of the compounds on the replication of herpes simplex viruses, types 1 and 2 (HSV-1 and HSV-2), as well as acyclovir-resistant herpes simplex viruses.

In the examples hereinafter, the inhibitory effect on herpes ribonucleotide reductase is noted for exemplary compounds of formula 1. Noteworthy, in the connection with this specific inhibition of herpes ribonucleotide reductase, is the relatively minimal effect or absence of such an effect of the compounds on cellular ribonucleotide reductase activity required for normal cell replication.

A method for demonstrating the inhibitory effect of the compounds of formula 1 on viral replication is the cell

culture technique; see, for example, T. Spector et al., Proc. Natl. Acad. Sci. USA, 82, 4254 (1985).

The therapeutic effect of the compounds of formula 1 can be demonstrated in laboratory animals, for instance, by using an assay based on the murine model of herpes simplex virus-induced ocular disease for antiviral drug testing, described by C.R. Brandt et al., J. Virol. Meth., 36, 209 (1992).

When a compound of this invention, or one of its therapeutically acceptable acid addition salts, is employed as an antiviral agent, it is administered topically or systemically to warm-blooded animals, e.g. humans, pigs or horses, in a vehicle comprising one or more pharmaceutically acceptable carriers, the proportion of which is determined by the solubility and chemical nature of the compound, chosen route of administration and standard biological practice. For topical administration, the compound can be formulated in pharmaceutically accepted vehicles containing 0.1 to 5 percent, preferably 0.5 to 5 percent, of the active agent. Such formulations can be in the form of a solution, cream or lotion.

For systemic administration, the compound of formula 1 is administered by either intravenous, subcutaneous or intramuscular injection, in compositions with pharmaceutically acceptable vehicles or carriers. For administration by injection, it is preferred to use the compounds in solution in a sterile aqueous vehicle which may also contain other solutes such as buffers or preservatives as well as sufficient quantities of pharmaceutically acceptable salts or of glucose to make the solution isotonic.

Suitable vehicles or carriers for the above noted formulations are described in standard pharmaceutical texts, e.g. in "Remington's Pharmaceutical Sciences", 18th ed, Mack Publishing Company, Easton, Penn., 1990.

The dosage of the compound will vary with the form of administration and the particular active agent chosen. Furthermore, it will vary with the particular host under treatment. Generally, treatment is initiated with small increments until the optimum effect under the circumstances is reached. In general, the compound is most desirably administered at a concentration level that will generally afford antivirally effective results without causing any harmful or deleterious side effects.

With reference to topical application, the compound of formula 1 is administered cutaneously in a suitable topical formulation to the infected area of the body e.g. the skin or part of the oral or genital cavity, in an amount sufficient to cover the infected area. The treatment should be repeated, for example, every four to six hours until lesions heal.

With reference to systemic administration, the compound of formula 1 is administered at a dosage of 10 mg to 150 mg per kilogram of body weight per day, although the aforementioned variations will occur. However, a dosage level that is in the range of from about 10 mg to 100 mg per kilogram of body weight per day is most desirably employed in order to achieve effective results.

Another aspect of this invention comprises a cosmetic composition comprising a herpes viral prophylactic amount of the compound of formula 1, or a therapeutically acceptable salt thereof, together with a physiologically acceptable cosmetic carrier. Additional components, for example, skin softeners, may be included in the formulation. The cosmetic formulation of this invention is used prophylactically to prevent the outbreak of herpetic lesions of the skin. The formulation can be applied nightly to susceptible areas of the skin. Generally, the cosmetic composition contains less of the compound than corresponding pharmaceutical compositions for topical application. A preferred range of the amount of the compound in the cosmetic composition is 0.5 to 5 percent by weight.

Although the formulations disclosed hereinabove are indicated to be effective and relatively safe medications for treating herpes viral infections, the possible concurrent administration of these formulations with other antiviral medications or agents to obtain beneficial results is not excluded. Such other antiviral medications or agents include the antiviral nucleosides, for example, acyclovir, and antiviral surface active agents or antiviral interferons such as those disclosed by S.S. Asculai and F. Rapp in U.S. patent 4,507,281, March 26, 1985.

More specifically with respect to treating herpes viral infections by concurrent administration, it has been found that the antiherpes activity of an antiviral nucleoside analogs can be enhanced synergistically, without the concomitant enhancement of toxic effects, by combining the same with a compound of formula 1. Accordingly, there is provided herewith a pharmaceutical composition for treating herpes infections in a mammal comprising a pharmaceutically acceptable carrier, and an effective amount of the combination of an antiviral nucleoside analog or a therapeutically acceptable salt thereof, and a ribonucleotide reductase inhibiting compound of formula 1 or a therapeutically acceptable salt thereof.

The antiviral nucleoside analog employed in the combination is one which is enzymatically convertible *(in vivo)* to a viral DNA polymerase inhibitor of, and/or an alternative substrate for, a herpes DNA polymerase. The antiviral nucleoside analog can be selected from known nucleoside analogs. Preferred nucleoside analogs of the invention include acyclovir and its analogs; for example, the compounds of formula 19

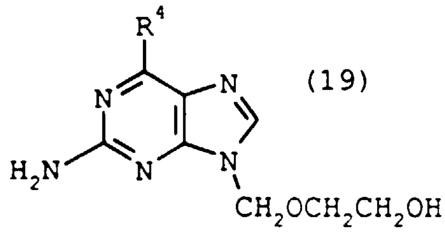

(19)

wherein $R^4$ is hydrogen, hydroxy or amino, or a therapeutically acceptable salt thereof. (Formula 19 wherein $R^4$ is hydroxy represents acyclovir.)

Other preferred antiviral nucleoside analogs for use according to the present invention include penciclovir, famciclovir and valacyclovir.

An example of a therapeutically acceptable salt of the nucleoside analogs is the sodium salt.

The term "synergistic effect" when used in relation to the antiviral or antiherpes activity of the above defined combination of the nucleoside analog and the compound of formula 1 means an antiviral or antiherpes effect which is greater than the predictive additive effect of the two individual components of the combination.

When utilizing the combination of this invention for treating herpes infections, the combination is administered to warm blooded animals, e.g. humans, pigs or horses, in a vehicle comprising one or more pharmaceutically acceptable carriers, the proportion of which is determined by the solubility and chemical nature of the nucleoside analog and the compound of formula 1, chosen route of administration, standard biological practice, and by the relative amounts of the two active ingredients to provide a synergistic antiviral effect. The combination may be administered topically. For example, the two active agents (i.e. the antiviral nucleoside analog and the compound of formula 1, or their therapeutically acceptable salts) can be formulated in the form of solutions, emulsions, creams, or lotions in pharmaceutically acceptable vehicles. Such formulation can contain 0.01 to 1.0 percent by weight of the nucleoside analog, or a therapeutically acceptable salt thereof, and about 0.05 to 1 percent by weight of the compound of formula 1, or a therapeutically acceptable salt thereof.

In any event, the two active agents are present in the pharmaceutical composition in amounts to provide a synergistic antiherpes effect.

The following examples illustrate further this invention. Temperatures are given in degrees Celsius. Solution percentages express a weight to volume relationship, and solution ratios express a volume to volume relationship, unless stated otherwise. Nuclear magnetic resonance (NMR) spectra were recorded on a Bruker 400 MHz spectrometer; the chemical shifts (δ) are reported in parts per million. Abbreviations used in the examples include Boc: tert-butyloxycarbonyl; Bzl: benzyl; DMSO: dimethylsulfoxide; Et: ethyl; EtOH: ethanol; EtOAc: ethyl acetate; $Et_2O$: diethyl ether; HPLC: high performance liquid chromatography; Me: methyl; MeOH: methanol; Pr: propyl; TLC: thin layer chromatography; THF: tetrahydrofuran.

<u>Example 1</u>

General Procedure for Coupling Reactions

{See also R. Knorr et al., Tetrahedron Letters, <u>30</u>, 1927 (1989).}

The first reactant, i.e. a free amine (or its hydrochloride salt), is dissolved in $CH_2Cl_2$ or $CH_3CN$ and the solution is cooled to 4°. Under a nitrogen atmosphere, four equivalents of N-methylmorpholine are added to the stirred solution. After 20 min, one equivalent of the second reactant, i.e. a free carboxylic acid, and 1.05 equivalents of the coupling agent are added. (Practical and efficient coupling reagents for this purpose are (benzotriazol-1-yloxy)tris-(dimethylamino)phosphonium hexafluorophosphate or preferably 2-(1H-benzotriazol-1-yl)-N,N,N',N'-tetramethyluronium tetrafluoroborate. The reaction is monitored by TLC. After completion of the reaction, the solvent is evaporated under reduced pressure. The residue is dissolved in EtOAc. The solution is washed successively with 1 N aqueous citric acid, 10% aqueous $Na_2CO_3$ and brine. The organic phase is dried ($MgSO_4$), filtered and concentrated under reduced pressure. The residue is purified on silica gel ($SiO_2$) according to Still's flash chromatography technique (W.C. Still et al., J. Org. Chem., <u>43</u>, 2923 (1978)).

Example 2

Preparation of 1(R) -Ethyl-2,2-dimethylpropylamine Hydrochloride ($NH_2$- (R)-CH (Et) $CMe_3$.HCl).

To a cooled solution (0°) of 4,4-dimethyl-3-pentanone (106 g, 0.928 mol) and (R)-α-methylbenzylamine (111 g, 0.916 mol) in benzene (1 L), a solution of $TiCl_4$ (50.5 mL, 0.461 mol) in benzene (200 mL) was added at a rate that kept the temperature of the mixture below 10°. Thereafter, the mixture was stirred mechanically for 3 h at 40°, cooled to room temperature and filtered through diatomaceous earth. The diatomaceous earth was washed with $Et_2O$. The combined filtrate and wash was concentrated. The residue was dissolved in dry MeOH (2 L). The solution was cooled to 0° and $NaBH_4$ (20 g, 0.53 mol) was added portionwise while maintaining the temperature of the mixture below 5°. The methanol was evaporated. The residue was dissolved in $Et_2O$. The solution was washed with brine, dried ($MgSO_4$) and concentrated to give a reddish oil (a 18:1 mixture of diastereoisomers as indicated by NMR). The oil was purified by flash chromatography ($SiO_2$, eluent: EtOAc/hexane, 7:93) to afford N-(1(R)-phenylethyl)-1(R)-ethyl-2,2-dimethylpropylamine as a liquid (110 g, 54% yield). This material was dissolved in hexane (1.5 L). 1 N HCl in $Et_2O$ (550 mL) was added to the solution over a period of 15 min. The resulting white solid was collected on a filter and then washed with hexane to provide N-(1(R)-phenylethyl)-1(R)-ethyl-2,2-dimethylpropylamine hydrochloride (125 g, 97% yield). $^1H$ NMR($CDCl_3$) δ 7.79-7.74 (m, 2H), 7.48-7.30 (m, 3H), 4.49-4.31 (m, 1H), 2.44-2.36 (m, 1H), 2.23 (d, J = 6.5 Hz, 3H), 1.95-1.54 (m, 2H), 1.14 (s, 9H), 0.55 (t, J = 7.5 Hz, 3H).

A solution of the latter compound (41.5 g) in MeOH (120 mL) was mixed with 10% Pd/C (*w*/*w*) (4.2 g) and the mixture was shaken under 50 psi of hydrogen in a Parr hydrogenator at room temperature for 48 h. The mixture was filtered through diatomaceous earth and the filtrate was concentrated to give the desired $NH_2$-(R)-CH(Et)$CMe_3$ in the form of its hydrochloric acid addition salt, as a white solid (25 g, 100% yield). $^1H$ NMR($CDCl_3$) δ 8.40-8.10 (broad s, 3H), 2.85-2.70 (m, 1H), 1.90-1.58 (m, 2H), 1.22 (t, J = 7 Hz, 3H), 1.10 (s, 9H).

Example 3

Preparation of the Intermediate H-Asp(cyPn) (Bzl)-NH-(R)-CH(Et)$CMe_3$ (the compound of formula 13 wherein $R^4$ is $NH_2$ and $W^3$ is Bzl)

(a) (S)-α-Azido-1-{(phenylmethoxy)carbonyl}cyclopentaneacetic acid (the compound of formula 10 wherein $W^3$ is Bzl): This compound was prepared from 2-oxospiro[4.4]nonane-1,3-dione, described by M.N. Aboul-Enein et al., Pharm. Acta Helv., 55, 50 (1980), according to the asymmetric azidation method utilizing the Evan's auxiliary; see D.A. Evans et al., J. Amer. Chem. Soc., 112, 4011 (1990).

More explicitly, a 1.6 M hexane solution of butyllithium (469 mL, 750 mmol) was added dropwise under an argon atmosphere to a solution of the chiral auxiliary, 4(S)-(1-methylethyl)-2-oxazolidinone, (96.8 g, 750 mmol) (described by L. N. Pridgen and J. Prol., J. Org, Chem, 54, 3231 (1989)} in dry THF at -40°. The mixture was stirred at -40° for 30 min and then cooled to -78°. 2-Oxospiro[4.4]nonane-1,3-dione was added dropwise to the cooled mixture. The mixture was stirred at 0° for 1 h. Thereafter, a 20% aqueous solution of citric acid (600 mL) was added to the mixture. The organic phase was separated and the aqueous phase was extracted with EtOAc. The combined organic phases were washed with brine, dried ($MgSO_4$) and concentrated under reduced pressure to give 3-[2-(1-carboxycyclopentyl)-1-oxoethyl)}-4(S)-(1-methylethyl)-2-oxazolidinone as a pink solid (300 g).

The latter solid (ca 750 mmol) was dissolved in $CH_3CN$ (1 L). Benzyl bromide (89.2 mL, 750 mmol) and 1,8-diazabicyclo [5.4.0]undec-7-ene (112 mL, 750 mmol) were added to the solution. The mixture was stirred under argon for 16 h. The volatiles were removed under reduced pressure. The residue was dissolved in $H_2O$/EtOAc. The organic phase was separated, washed with a 10% aqueous solution of citric acid and brine, dried ($MgSO_4$) and concentrated under reduced pressure to give an oil. Crystallization of the oil from hexane/EtOAc gave the corresponding benzyl ester as a white solid (204 g, 73% yield).

A solution of the latter compound (70 g, 190 mmol) in dry THF (200 mL) was cooled to -78°. A 0.66 M THF solution of potassium bis(trimethylsilyl)amide (286 mL, 190 mmol) containing 6% cumene was added over a period of 15 min to the cooled solution. The mixture was stirred at -78° for 45 min. A solution of 2,4,6-triisopropylbenzenesulfonyl azide (67 g, 220 mmol) in dry THF (100 mL) was added in one portion to the cold mixture, followed two minutes later by the addition of glacial acetic acid (50 mL, 860 mmol). The mixture was warmed and stirred at 35-45° for 1 h. The volatiles were removed under reduced pressure. The yellow residue was triturated with hexane/EtOH (4:1, 1.7 L). The resulting white solid was collected on a filter. The filtrate was mixed with $SiO_2$ (230-240 mesh). Volatiles were removed under reduced pressure and the residual solid was dried at 35° under reduced pressure to remove cumene. The residual solid then was placed on a column of $SiO_2$. Elution of the column with

hexane-EtOAc (9:1) and concentration of the eluent gave 3-{{2(S)-azido-1-oxo-2-{1-{(phenylmethoxy)carbonyl) cyclopentyl}-ethyl)-4(S)-(1-methylethyl)-2-oxazolidinone (66 g, 86% yield).

A solution of the latter compound (13.4 g, 32.4 mmol) in THF/$H_2O$ (3:1, 608 mL) was cooled to 0°. Hydrogen peroxide/$H_2O$ (3:7, 16.3 mL, 141 mmol of $H_2O_2$) was added to the cooled solution, followed by the addition of LiOH.$H_2O$ (2.86 g, 68.2 mmol). The mixture was stirred at 0° for 45 min and then quenched with a 10% aqueous solution of sodium sulfite (400 mL). After $NaHCO_3$ (1.93 g) had been added, the mixture was concentrated under reduced pressure. The chiral auxiliary was recovered by continuous extraction (aqueous $NaHCO_3$/chloroform) for 20 h. Thereafter, the aqueous phase was cooled to 0° rendered acidic by the addition of concentrated HCl and then extracted with EtOAc. The extract was washed with brine, dried ($MgSO_4$) and concentrated under reduced pressure to give (S)-α-azido-1-{(phenylmethoxy)carbonyl}cyclopentaneacetic acid as a white solid (8.2 g, 84% yield). $^1$H NMR ($CDCl_3$) δ 7.40-7.28 (m, 5H), 5.12 (s, 2H), 4.55 (s, 1H), 2.30-2.20 (m, 1H), 2.05-1.95 (m, 2H), 1.8-1.6 (m, 5H).

(b) The title compound of this example: By following the coupling procedure of example 1 and using the hydrogen chloride salt of $NH_2$-(R)-CH(Et)$CMe_3$ of example 2 as the first reactant and (S)-α-azido-1-{(phenylmethoxy)carbonyl}cyclopentaneacetic acid of section (a) of this example as the second reactant, N-{1(R)-ethyl-(2,2-dimethylpropyl)}-(S)-α-azido-1-{(phenylmethoxy)carbonyl}cyclopentaneacetamide was obtained. Reduction of the latter compound with tin(II) chloride in MeOH according to the method of N. Maiti et al., Tetrahedron Letters, 27, 1423 (1986), followed by purification by chromatography ($SiO_2$, hexane - $Et_2O$, 1:1), gave the title compound of this example. $^1$H NMR ($CDCl_3$) δ 7.36-7.27 (m, 5H), 7.08 (d, J = 10.5 Hz, 1H), 5.17 (d, J = 12.3 Hz, 1H), 5.09 (d, J = 12.3 Hz, 1H), 3.72 (s, 1H), 3.56 (ddd, J = 10.5, 10.5, 2.5 Hz, 1H), 2.23-1.15 (m, 2H), 1.87-1.80 (m, 1H), 1.76-1.57 (m, 8H), 1.17-1.03 (m, 1H), 0.88 (s, 9H) and 0.86 (t, J = 7.3 Hz, 3H).

Example 4

Preparation of the Intermediate Boc-Tbg-$CH_2$-(R)-CH($CH_2$C(O)$CMe_3$)C(O)OBzl (the compound of formula 2 wherein $W^1$ is Boc and $W^2$ is Bzl)

(a) Boc-Tbg-OMe (the compound of formula 3 wherein $W^1$ is Boc) : A solution of Boc-Tbg-OH (68 g, 0.30 mol) in dry $CH_3CN$ (0.5 L) was cooled to 0°. 1,8-Diazabicyclo[5.4.0]undec-7-ene (54 mL, 0.36 mol) was added over a period of 10 min to the cooled solution, followed by the addition of $CH_3I$ (37 mL, 0.60 mol). The reaction mixture was stirred at room temperature (20-22°) for 4 h and then concentrated under reduced pressure. The residue was partitioned between EtOAc and $H_2O$. The organic phase was washed with $H_2O$, an aqueous saturated solution of $NaHCO_3$ (2 X), and brine. Thereafter, the organic phase was dried ($MgSO_4$) and concentrated to afford a clear viscous liquid. This material was distilled bulb to bulb (oil pump vacuum, air bath temperature at 110°) to provide the desired product as a colorless oil (65 g, 88% yield). $^1$H NMR ($CDCl_3$) 6 5.10 (broad d, J = 9.0 Hz, 1H), 4.10 (d, J = 9.0 Hz, 1H), 3.72 (s, 3H), 1.44 (s, 9H), 0.96 (s, 9H).

(b) Boc-Tbg-$CH_2$-P(O) $(OMe)_2$ (the compound of formula 5 wherein $W^1$ is Boc) : At -78° under a nitrogen atmosphere, a 5 L flask equipped with a mechanical stirrer, an addition funnel with jacket and a thermometer was charged with a solution of BuLi in hexane (3.60 mol, 361 mL of a 10 N solution). A cold (-78°) solution of freshly distilled dimethyl methylphosphonate (391 mL, 3.60 mol) in dry THF (1 L) was added dropwise *via* the addition funnel over a 1 h period. The mixture was stirred at -78° for 30 min. A cold (-78°) solution of Boc-Tbg-OMe (111 g, 0.452 mol) in THF (0.5 L) was added dropwise over a 20 min period. The reaction was stirred at -78° for 45 min, and then allowed to warm to about -30° over a 30 min period. Following the sequential addition of glacial acetic acid (0.25 L) and $H_2O$ (0.3 L), the mixture was extracted with EtOAc (1 L). The organic layer was washed with $H_2O$, a 10% aqueous solution of $NaHCO_3$ and brine, dried ($MgSO_4$) and concentrated. The resulting solid was triturated with hexane to give the desired phosphonate as a white powder with mp 84-86° (144 g, 95% yield). $^1$H NMR ($CDCl_3$) δ 5.23 (broad d, J = 9.0 Hz, 1H), 4.25 (d, J = 9.0 Hz, 1H), 3.80 (d, J = 11.4 Hz, 6H), 3.30 (dd, J = 22.0, 14.6 Hz, 1H), 3.12 (dd, J = 22.0, 14.6 Hz, 1H), 1.44 (s, 9H), 1.00 (s, 9H).

The phosphonate is used in section (d) of this example.

(c) HC(O)C(O)OBzl (the compound of formula 6 wherein $W^2$ is Bzl) : Solid $H_5IO_6$ (49.3 g, 0.216 mol) was added portionwise to a solution of dibenzyl L-tartrate (70 g, 0.21 mol) in $Et_2O$ (900 mL). The mixture was stirred for 2.5 h at room temperature and then filtered. The filtrate was dried ($MgSO_4$) and concentrated. The residual syrup was dissolved in hexane-$Et_2O$ (2:3). The resulting milky solution was filtered through a pad of diatomaceous earth. The

pad was washed with hexane-$Et_2O$ (2:5). The combined filtrate and washing were concentrated to yield benzylglyoxylate as an oil (69.9 g, -90% yield). $H^1$ NMR ($CDCl_3$) showed a mixture of aldehyde and hydrate form. Characteristic chemical shifts: δ 9.25 (s), 7.87-7.21 (m, 5H), 5.47-5.03 (m), 4.56 (broad s).

(d) The γ-keto-α,β-unsaturated ester Boc-Tbg-(*E*)-CH=CHC(O)OBzl (the compound of formula 7 wherein $W^1$ is Boc and $W^2$ is Bzl): A solution of Boc-Tbg-$CH_2$-P(O)$(OMe)_2$ (121 g, 0.359 mol), described in section (b) of this example, and triethylamine (0.10 L, 0.72 mol) in $CH_3CN$ (0.7 L) was stirred under nitrogen for 10 min at room temperature. Thereafter, a solution of HC(O)C(O)OBzl (121 g, ~0.36 mol) in $CH_3CN$ (0.15 L) was added over 30 min. The mixture was stirred for 24 h and then concentrated. The residue was dissolved in $Et_2O$-hexane (2:1, 0.8 L) . The solution was washed with a 10% aqueous solution of citric acid, a saturated solution of $NaHCO_3$ and brine, dried ($MgSO_4$) and concentrated. The resulting orange oil was passed through a silica gel pad (12 x 10 cm) using EtOAc-hexane (3:20) as the eluent. Concentration of the eluate gave the desired γ-keto-α,β-unsaturated ester as a yellow oil (112 g, 83% yield). $^1H$ NMR ($CDCl_3$) δ 7.42-7.32 (m, 5H), 7.23 (d, J = 15.9 Hz, 1H), 6.80 (d, J = 15.9 Hz, 1H), 5.25 (s, 2H), 5.21 (broad d, J = 8.9 Hz, 1H), 4.43 (d, J = 8.9 Hz, 1H), 1.42 (s, 9H), 0.96 (s, 9H).

The γ-keto-α,β-unsaturated ester is used in section (f) of this example.

(e) $CH_2$=$CHCH_2OC(O)CH_2C(O)CMe_3$ (the compound of formula 8): A solution of lithium bis(trimethylsilyl)amide in THF (1 N, 0.8 L) was cooled to-78°. A solution of allyl acetate (39 mL, 0.36 mol) in THF (40 mL) was added dropwise to the cooled solution. The mixture was stirred at -78° for 1 h. Thereafter, a solution of trimethylacetyl chloride (47 mL, 0.38 mol) was added dropwise and the resulting mixture was stirred for 25 min at -78°. Hexane (0.3 L) and an aqueous solution of HCl (3 N, 0.6 L) were added to the mixture. The organic phase was separated and washed with a saturated aqueous solution of sodium bicarbonate, brine and water. The organic phase was dried ($MgSO_4$), and concentrated to afford an orange oil. Distillation (bulb to bulb, air bath temperature of 60°, 0.25 Tor.) of the crude product gave desired ester as a colorless oil (62 g, 92% yield). $^1H$ NMR ($CDCl_3$) δ 6.02-5.87 (m, 1H), 5.35 (broad d, J = 17.2 Hz, 1H), 5.25 (broad d, J = 9.5 Hz, 1H), 4.63 (broad d, J = 5.6 Hz, 2H), 3.59 (s, 2H), 1.19 (s, 9H).

(f) The Michael adduct, i.e. Boc-Tbg-$CH_2$-(R)-CH{CH(C(O)$CMe_3$) (C (O)$OCH_2CH$=$CH_2$)}C(O) OBzl (the compound of formula 9 wherein $W^1$ is Boc and $W^2$ is Bzl): Solid NaH (2.7 g of a 60% oil dispersion, 0.07 mol) was added over a 15 min period to a solution of $CH_2$=$CHCH_2OC(O)CH_2C(O)CMe_3$ (83.2 g, 0.452 mol) in THF (0.8 L). The reaction mixture was stirred at room temperature under an atmosphere of argon until all the solid dissolved (30 min). The homogeneous solution was cooled to -60° (solution temperature) and a solution of Boc-Tbg- (*E*) -CH=CHC (O) OBzl (170 g, 0.45 mol), described in section (d) of this example, in THF (0.5 L) was added slowly over a period of 45 min. Thereafter, the reaction mixture was stirred at -60° for 5 h. A 10% aqueous solution of citric acid was added and the mixture was allowed to warm to room temperature. The mixture was extracted with $Et_2O$. The organic phase was washed with a 5% aqueous solution of sodium bicarbonate and brine, dried ($MgSO_4$) and concentrated to afford an orange oil (250 g) which was used without further purification in the next reaction.

(g) Boc-Tbg-$CH_2$-(R)-CH ($CH_2C(O)CMe_3$)C(O)-OBzl: Pyrrolidine (56 mL, 0.54 mol) was added to a stirred solution of tetrakistriphenylphosphine palladium(O) (2.60 g, 2.25 mmol, 0.5% molar) in $CH_2Cl_2$ (250 mL) and $CH_3CN$ (250 mL) at 0° under an atmosphere of argon. The mixture was allowed to warm to room temperature. A solution of the Michael adduct from the preceding section (250 g, 0.45 mol) in $CH_2Cl_2$-$CH_3CN$ (200 mL:200 mL) was added to the mixture. After 3 h, the mixture was concentrated to yield an orange oil. The crude oil was dissolved in a mixture of $Et_2O$-hexane (1:1, 1L). The solution was washed with a 10% aqueous solution of citric acid, 10% aqueous solution of sodium bicarbonate, and brine, dried ($MgSO_4$) and concentrated to give the title compound of this example as an orange oil (203 g, >90% yield). This material was used without further purification in example 5. A small sample was purified by $SiO_2$ chromatography. Elution with hexane-EtOAc (9:1) gave the pure title compound as a colorless oil. $[\alpha]_D^{25}$ + 11.5 (c = 1.3, $CHCl_3$); $^1H$ NMR ($CDCl_3$) δ 7.38-7.28 (m, 5H), 5.10 (s, 2H), 5.07 (broad d, J = 9.2 Hz, 1H), 4.08 (d, J = 9.2 Hz, 1H), 3.38-3.31 (m, 1H), 3.09 (dd, J = 18.8, 6.0 Hz, 1H), 2.94 (dd, J = 18.4 6.1 Hz, 1H), 2.82 (dd, J = 18.4, 6.1 Hz, 1H), 2.77 (dd, J = 18.8, 6.0 Hz, 1H), 1.42 (s, 9H), 1.10 (s, 9H), 0.95 (s, 9H). The diastereoisomeric purity was assessed to be >35:1 by NMR; see P.L. Beaulieu et al., European patent application 560 267, published September 15, 1993. In order to assess the enantiomeric purity of the title compound, the Boc protective group ($W^1$) was removed with 4 N HCl in dioxane and the resulting amine was converted to a Mosher amide (see J.A. Dale et al., *vide supra)* . By comparing results from a product prepared by the procedure of this example with results obtained with a racemic mixture of the title compound, the enantiomeric excess for said product was determined to be >96% by NMR and >99% by chiral column chromatography. The latter determination was performed by normal phase HPLC on a Chiracel® OD column from Daicel Chemical Industries Limited, Tokyo, Japan (US distributor: Chiral Technologies Inc., Exton PA, USA). EtOH-hexane (1:19) was the eluent

and UV detection at 215 nm was employed.

Example 5

Preparation of the Intermediate Boc-Tbg-$CH_2$-(R)-CH($CH_2$C(O)$CMe_3$) C(O)OH (the compound of formula 14 wherein $W^1$ is Boc)

To a solution of the title compound of example 4 (171 g, 0.36 mol) in EtOH (1.4 L) was added 10% Pd/C (10 g). The resultant mixture was stirred vigorously under one atmosphere of hydrogen for 5 h. Thereafter, the reaction mixture was subjected to filtration through diatomaceous earth. The filtrate was concentrated under reduced pressure. The residue was dissolved in a saturated aqueous solution of $Na_2CO_3$. The aqueous solution was washed with hexane-$Et_2O$ (8:2), rendered acidic with citric acid and extracted with EtOAc. The extract was dried ($MgSO_4$) and concentrated. The orange residue was dissolved in $Et_2O$ and the resulting solution was passed through a silica gel pad (12 x 12 cm). Concentration gave the title compound of this example as a solid with mp 62-65° (117 g, 84% yield). $^1H$ NMR ($CDCl_3$) δ 5.18 (d, J = 8.8 Hz, 1H), 4.09 (d, J = 8.8 Hz, 1H), 3.35-3.29 (m, 1H), 3.09 (dd, J = 18.8, 6.3 Hz), 2.94 (dd, J = 18.4, 6.3 Hz, 1H), 2.83 (dd, J = 18.4, 6.3 Hz, 1H), 2.78 (dd, 18.8, 6.3 Hz, 1H), 1.43 (s, 9H), 1.14 (s, 9H), 0.96 (s, 9H).

Example 6

Preparation of the Intermediate Boc-Tbg-$CH_2$-(R)-CH($CH_2$C(O)$CMe_3$)C(O)-Asp(cyPn)(Bzl)-NH-(R)-CH(Et)$CMe_3$ (the compound of formula 15 wherein $R^5$ is Boc and $W^3$ is Bzl)

By following the coupling procedure of example 1 and using the title compound of example 3 as the first reactant and the title compound of example 5 as the second reactant, the title compound of this example is obtained: $^1H$ NMR ($CDCl_3$) δ 7.43-7.26 (m, 6H), 6.76 (d, J = 10.0 Hz, 1H), 5.16 (s, 2H), 5.06 (d, J = 8.9 Hz, 1H), 4.62 (d, J = 8.9 Hz, 1H), 4.07 (d, J = 8.9 Hz, 1H), 3.60 (ddd, J = 10.0, 10.0, 2.5 Hz, 1H), 3.18-2.83 (m, 3H), 2.70 (dd, J = 16.9, 4.1 Hz, 1H), 2.68-2.54 (m, 1H), 1.90-1.52 (m, 9H), 1.42 (s, 9H), 1.11 (s, 9H), 0.94 (s, 9H), 0.88 (s, 9H), 0.78 (t, J = 7.3 Hz, 3H).

Example 7

Preparation of (1α,2α,6α)-2,6-dimethyl-1-isocyanatocyclohexane (the compound of formula 17 wherein $R^1$ and $R^3$ is methyl and $R^2$ is hydrogen)

(1α,2α,6α)-2,6-dimethyl-1-cyclohexanamine hydrochloride was prepared as follows: 2,6-Dimethylphenol was subjected to hydrogenation in the presence of Rh-$Al_2O_3$, followed by oxidation of the resulting 2,6-dimethylcyclohexanol isomers with chromic acid according to the method of I.J. Borowitz et al., J. Org. Chem., 37, 581 (1972) to obtain a mixture of *cis* and *trans* isomers of 2,6-dimethylcyclohexanone. The latter isomeric mixture of ketones was converted to a corresponding mixture of oximes, which was separated by chromatography on $SiO_2$. The desired cis isomer of 2, 6-dimethylcyclohexanone oxime was reduced (platinum black, 50 psi of hydrogen in a Parr hydrogenator glacial acetic acid, hydrochloric acid, 14 h) to give the desired (1α,2α,6α)-2,6-dimethyl-1-cyclohexanamine as its hydrochloric acid addition salt, mp >280 °C. The method for preparing and reducing the oximes to amines has been described previously by G. Bellucci et al., Gazz. Chim. Ital., 99, 1217 (1969).

The latter hydrochloric acid addition salt (3.11 g, 19.0 mmol) was suspended in toluene (100 mL) and a solution of phosgene in toluene (1.93 M, 4.9 mL, 95 mmol) was added. The mixture was heated at reflux for 2 h and then concentrated under reduced pressure to give the title compound as a clear colorless oil. This material was used as such in the following example.

In the same manner, other requisite substituted isocyanatocyclohexanes can be prepared. For example, (1α, 2α,6α)-2,6-diethyl-1-isocyanatocyclohexane was prepared from 2,6-diethylphenol *via* (1α,2α,6α)-2,6-diethyl-1-cyclohexanamine hydrochloride, mp >280°, and (1α,2α,4α,6α)-2,4,6-trimethyl-1-isocyanatocyclohexane was prepared from 2,4,6-trimethyl phenol *via* (1α,2α,4α,6α)-2,4,6-trimethyl-1-cyclohexanamine hydrochloride, mp >280°.

Example 8

Preparation of {{(1α,2α,6α)-2,6-Dimethyl-1-cyclohexanamino}carbonyl}-Tbg-$CH_2$-(R)-CH($CH_2$C(O)$CMe_3$)C(O)-Asp (cyPn) (Bzl)-NH-(R)-CH(Et)$CMe_3$ (the compound of formula 1 wherein $R^1$ and $R^3$ are methyl and $R^2$ is hydrogen)

To a solution of the title compound of example 6 (11.17 g, 15.05 mmol) in $CH_2Cl_2$ (20 mL) was added 4 M HCl/dioxane (100 mL). The mixture was stirred at room temperature for 30 min and then concentrated under reduced

pressure to give H-Tbg-$CH_2$-(R)-CH($CH_2$C(O)$CMe_3$)C(O)-Asp(cyPn)(Bzl)-NH-(R)-CH(Et)$CMe_3$ in the form of its hydrochloric acid addition salt. A suspension of the latter salt in dry $CH_2Cl_2$ (100 mL) was cooled to 0°. A solution of (1α, 2α, 6α)-2,6-dimethyl-1-isocyanatocyclohexane (-19 mmol), prepared as described in example 7, in dry $CH_2Cl_2$ (25 mL) was added to the cooled suspension. The mixture was stirred at 0° for 5 min. N-Methylmorpholine (3.3 mL, 30 mmol) was added to the mixture. Thereafter, the mixture was stirred at room temperature for 16 h and then concentrated under reduced pressure. The resulting residue was partitioned between EtOAc and a 5% aqueous solution of $Na_2CO_3$. The organic phase was washed with 1 N aqueous HCl and brine, dried ($MgSO_4$) and concentrated under reduced pressure. The resulting crude product was purified by flash chromatography ($SiO_2$, eluent:EtOAc/hexane, 1.5-2.5:10) to provide the corresponding benzyl ester of the title compound as a foam (9.59 g, 90% yield, after being dried to constant weight in high vacuum). This product was used as such for the following hydrogenolysis reaction.

The latter benzyl ester (9.59 g, 13.6 mmol) was subjected to hydrogenolysis (10% Pd/C (1.0 g), 1 atmosphere of $H_2$, absolute EtOH (150 mL), 2.5 h). The reaction mixture was filtered through a glass microfiber filter. The filtrate was concentrated under reduced pressure to 1/3 of its initial volume. This concentrate was filtered through a 0.4 µm membrane. The resulting filtrate was concentrated and the residue was crystallized (2 X) from EtOH-water to give the title compound as a white solid that was dried at 98° under high vacuum for 48 h (6.95 g, 85% yield). Mp 158-160°; $^1$H NMR ($d_6$-DMSO) δ 8.24 (d, J = 10 Hz, 1H), 6.93 (d, J = 10 Hz, 1H), 6.32 (d, J = 8.5Hz, 1H), 5.82 (d, J = 10 Hz, 1H), 4.93 (d, J = 10 Hz, 1H), 4.00 (d, J = 8.5 Hz, 1H), 3.67-3.63 (m, 1H), 3.45-3.37 (m, 1H), 3.23-3.15 (m, 1H), 2.78-2.59 (m, 4H), 2.08-2.01 (m, 1H), 1.70-1.47 (m, 13H), 1.34-1.19 (m, 4H), 1.03 (s, 9H), 0.93 (s, 9H), 0.87 (s, 9H), 0.74 (d, J = 6.5 Hz, 3H), 0.72 (d, J = 6.5 Hz, 3H), 0.64 (t, J = 7 Hz, 3H), FAB MS (m/z):705.4 $(M+H)^+$; Anal.: calculated for $C_{39}H_{68}N_4O_7$: C, 66.54; H, 10.01; N, 7.79; found, C, 66.18; H, 9.96; N, 7.88.

By following the procedure of this example but replacing (1α,2α, 6α)-2,6-dimethyl-1-isocyanatocyclohexane with (1α,2α,6α)-2,6-diethyl-1-isocyanatocyclohexane, then {{(1α,2α,6α)-2,6-diethyl-1-cyclohexanamino)carbonyl)-Tbg-$CH_2$-(R)-CH($CH_2$C(O)$CMe_3$)-C(O)-Asp(cyPn)-NH-(R)-CH(Et)$CMe_3$ was obtained: FAB/MS (m/z) : 733.7 $(M+H)^+$.

By following the procedure of this example but replacing (1α,2α,6α)-2,6-dimethyl-1-isocyanatocyclohexane with an equivalent amount of (1α,- 2α,4α,6α)-2,4,6-trimethyl-1-isocyanatocyclohexane, then {{(1α,2α,4α,6α)-2,4,6-trimethyl-l-cyclohexanamino}carbonyl)-Tbg-$CH_2$-(R)-CH($CH_2$C(O)$CMe_3$)C(O)-Asp(cyPn)-NH-(R)-CH(Et)$CMe_3$ was obtained; FAB/MS (m/z): 719.8 $(M+H)^+$.

Example 9

Inhibition of Herpes Simplex Virus (HSV-1) Ribonucleotide Reductase

a) Preparation of Enzyme

HSV-1 ribonucleotide reductase (partially purified) was obtained from quiescent BHK-21/C13 cells infected with strain F HSV-1 virus at 10 plaque forming units/cell as described by E.A. Cohen et al., J. Gen. Virol., 66, 733 (1985).

b) Assay

The assay described by P. Gaudreau et al., J. Biol, Chem., 262, 12413 (1987), is used to evaluate the capability of the compounds of formula 1 to inhibit HSV-1 ribonucleotide reductase activity. The assay results are expressed as the concentration of the compound producing 50% of the maximal inhibition ($IC_{50}$) of enzyme activity. The number of units of the enzyme preparation used in each assay was constant, based on the specific activity of the enzyme preparation. The results are relative to the activity obtained in control experiments without the test compound and represent the means of four assays that varied less than 10% with each other.

The following TABLE I illustrates the assay results obtained for exemplified compounds of formula 1.

TABLE I

| Compound of the Formula | $IC_{50}$ µM |
|---|---|
| 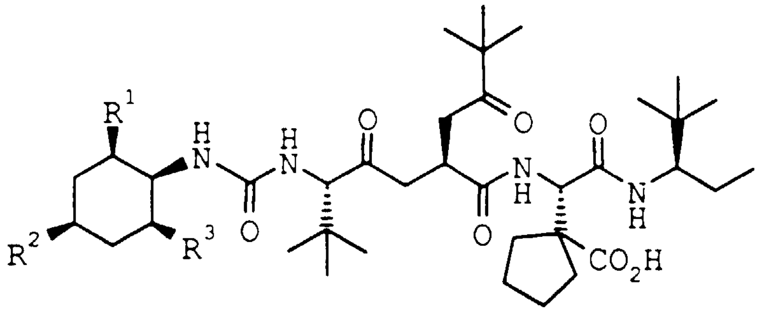 wherein $R^1$, $R^2$ and $R^3$ are as designated herein below | |
| $R^1$ and $R^3$ are methyl and $R^2$ is hydrogen | 0.095 |
| $R^1$ and $R^3$ are ethyl and $R^2$ is hydrogen | 0.110 |
| $R^1$, $R^2$ and $R^3$ are methyl | 0.130 |

Example 10

Inhibition of Herpes Simplex Virus (HSV-2) Replication in Cell Culture

Assay:

BHK-21/C13 cells (ATCC CCL 10) are incubated for two days in 150 $cm^2$ T-flasks (1.5 x $10^6$ cells/flask) with alpha-MEM medium (Gibco Canada Inc., Burlington, Ontario, Canada) supplemented with 8% (v/v) fetal bovine serum (FBS, Gibco Canada Inc.). The cells are trypsinized and then transferred to fresh media in a 24 well plate to give 2.5 x $10^5$ cells in 750 µL of media per well. The cells are incubated at 37° for a period of 6 h to allow them to adhere to the plate. Thereafter, the cells are washed once with 500 µL of alpha-MEM supplemented with 0.5% (v/v) FBS and then incubated with 750 µL of the same media (low serum) for 3 days. After this period of serum starvation, the low serum medium is removed and the cells are incubated in 500 µL of BBMT for 2 to 3 h. {BBMT medium is described by P. Brazeau et al., Proc. Natl. Acad. Sci. USA, 79, 7909 (1982).} Thereafter, the cells are infected with HSV-2 (multiplicity of infection = 0.02 PFU/cell) in 100 µL of BBMT medium. (Note: The HSV-2 used was strain HG-52, see Y. Langelier and G. Buttin, J. Gen. Virol., 57, 21 (1981); the virus was stored at -80°.) Following 1 h of virus adsorption at 37°, the media is removed and the cells are washed with BBMT (3 X 250 µL). The cells in each well are incubated with or without 200 µL of appropriate concentrations of the test agent in BBMT medium. After 28 h of incubation at 37°, the infected cells are harvested by first freezing the plate at -80°, followed by thawing. The cells in each well are scraped off the surface of the well with the help of the melting ice fragments. After complete thawing, the cell suspensions are collected and each well is rinsed with 150 µL of BBMT medium. The viral sample (suspension plus washing) is sonicated gently for 4 min at 4°. Cell debris are removed by centrifugation (1000 times gravity for 10 min at 4°). The supernatant is collected and stored at -80° until determination of viral titer.

Viral titration was performed by a modification of the colorimetric assay method of M. Langlois et al., Journal of Biological Standardization, 14, 201 (1986), and the application of the modified method to this cell culture assay is described in detail by R. Déziel and Y. Guindon, *vide supra.*

Accordingly, the percentage of virus growth inhibition can be determined for the various concentrations of the test agent. From this data, the $EC_{50}$, i.e. the concentration of the test agent effecting a 50% inhibition of virus replication, can be calculated.

Results:

The following TABLE II provides examples of the results obtained when compounds of formula 1 were evaluated according to the cell culture assay (HSV-2) of this example.

TABLE II

| Compound of the formula | $EC_{50}$ µM |
|---|---|
| 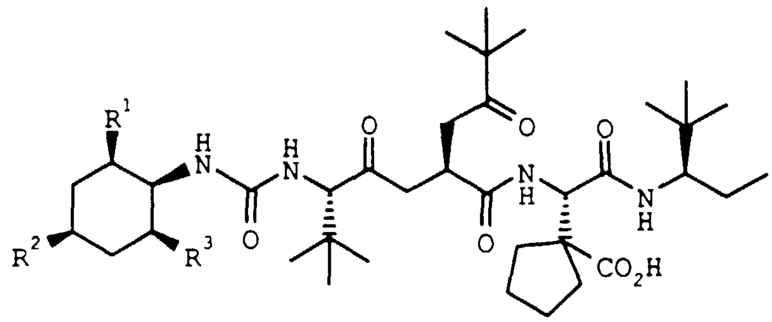<br>wherein $R^1$, $R^2$ and $R^3$ are as designated herein below | |
| $R^1$ and $R^3$ are methyl and $R^2$ is hydrogen | 7 |
| $R^1$ and $R^3$ are ethyl and $R^2$ is hydrogen | 5 |
| $R^1$, $R^2$ and $R^3$ are methyl | 6 |

Example 12

Synergistic Combinations

The synergistic action between the title compound of example 8 and acyclovir (ACV) against HSV-1 and HSV-2 was demonstrated by evaluating the two agents, each alone and then in various combinations in the cell culture assay, using strains of HSV-1 or HSV-2 and applying the isobole method to the results obtained in these studies; see J. Sühnel, J. Antiviral Research, 13, 23 (1990) for a description of the isobole method.

More explicitly with reference to the isobole method, this method requires experimental data generated for the two test compounds, each alone and in different combinations. In this way selected concentrations of the title compound of example 8 ($EC_5$, $EC_{10}$, $EC_{20}$ and $EC_{30}$) were added to a given concentration of ACV and the $EC_{50}$'s were evaluated as described previously. For these experiments, the $EC_5$, $EC_{10}$, $EC_{20}$ and $EC_{30}$ of the title compound of example 8 (i.e. the test compound) were derived from inhibition curves previously obtained. An isobologram is generated using a value termed $FIC_{60}$ (ACV) (which is the ratio of the concentration of ACV required to inhibit HSV replication by 60% in the presence of a fixed concentration of the test compound to the concentration required in the absence of the test compound) . This is plotted against a term representing the ratio of the fixed concentration of the test compound to the concentration of the test compound that reduced 60% inhibition of HSV replication in the absence of ACV.

Equations:

*X axis:*

$$\frac{\text{[the fixed concentration of the test compound added]}}{EC_{60}\text{ of the test compound alone}}$$

*Y axis:*

$$FIC_{60}\ (ACV) = \frac{EC_{60}\ (ACV + X\ \mu M\ of\ the\ test\ compound}{EC_{60}\ (ACV\ alone)}$$

The following TABLES III and IV are illustrative of results obtained when combinations of ACV and the title compound of example 8 (TC) were evaluated for their antiherpes activity against HSV-1 and HSV-2.

The virus strains and their multiplicity of infections (MOI) employed were HSV-1 KOS strain (MOI = 0.01 PFU/cell) for the studies illustrated in TABLE III, and HSV-2 HG-52 strain (MOI = 0.02 PFU/cell) for the studies illustrated in TABLE IV.

TABLE III

| SYNERGISTIC STUDIES OF ACYCLOVIR (ACV) AND THE TITLE COMPOUND OF EXAMPLE 8 (TC) AGAINST HSV-1 | |
|---|---|
| COMPOUNDS | $EC_{50}$ (μM)[1] |
| Compound Alone | |
| ACV[2] | 2.2 |
| TC | 2.3 |
| Synergistic Studies | |
| ACV + 1.0 μM of TC | 0.70 |
| ACV + 1.2 μM of TC | 0.55 |
| ACV + 1.4 μM of TC | 0.50 |
| ACV + 1.6 μM of TC | 0.22 |
| ACV + 1.8 μM of TC | 0.16 |
| ACV + 2.0 μM of TC ($EC_{30}$) | 0.12 |

TABLE IV

| SYNERGISTIC STUDIES OF ACYCLOVIR (AC) AND THE TITLE COMPOUND OF EXAMPLE 8 (TC) AGAINST HSV-2 | |
|---|---|
| COMPOUNDS | $EC_{50}$ (μM) [1] |
| Compound Alone | |
| ACV [2] | 1.8 |
| TC | 5.2 |
| Synergistic studies | |
| ACV + 2 μM of TC | 0.28 |
| ACV + 3 μM of TC | 0.38 |
| ACV + 4 μM of TC ($EC_{30}$) | 0.16 |

(1) Stock solutions of the title compound of example 8 were filtered through a 0.22 μM membrane and then the concentration of the compound in the filtered solution was determined by HPLC.

(2) Acyclovir was obtained from Burroughs Wellcome Inc., Kirkland, Quebec, Canada.

Note: In the preceding studies of TABLES III and IV, the inhibition of the HSV replication was observed at concentrations significantly below the cytotoxic levels for the test compounds as determined by the cytotoxicity assay of F. Denizot and R. Lang, J. Immunol. Methods, 89, 271 (1986).

The results of TABLES III and IV show that, on combining the title compound of example 8 with acyclovir, a proportional lowering of the $IC_{50}$ of acyclovir is effected as the ratio of the concentrations of the title compound of example 8 is increased. Hence, these synergistic studies demonstrate that the compounds of formula 1 are able to potentiate the antiherpes activity of acyclovir against HSV-1 and HSV-2.

The results of TABLES III and IV are graphically illustrated in accompanying Figures 1 and Figures 2, respectively.

## Claims

**1.** A compound of formula 1

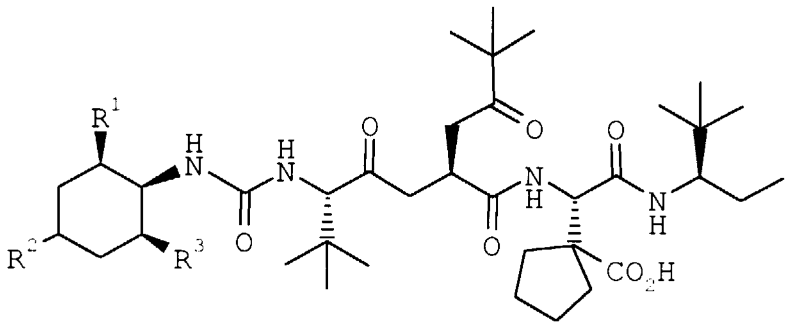

(1)

wherein $R^1$ is (1-3C)alkyl, $R^2$ is hydrogen or (1-3C)alkyl and $R^3$ is (1-3C)alkyl, or a therapeutically acceptable salt thereof.

**2.** A compound as defined in claim 1 wherein $R^1$ and $R^3$ are both methyl or both ethyl, and $R^2$ is hydrogen, methyl or ethyl, or a therapeutically acceptable salt thereof.

**3.** A compound as defined in claim 2 wherein $R^1$ and $R^3$ are both methyl and $R^2$ is hydrogen or a *cis*-methyl relative to $R^1$ and $R^3$; or $R^1$ and $R^3$ are both ethyl and $R^2$ is hydrogen; or a therapeutically acceptable salt thereof.

**4.** A pharmaceutical composition comprising an antiherpes virally effective amount of a compound as defined in claim 1, or a therapeutically acceptable salt thereof, and a pharmaceutically or veterinarily acceptable carrier.

**5.** A cosmetic composition comprising a compound as defined in claim 1, or a therapeutically acceptable salt thereof, and a physiologically acceptable carrier, suitable for topical application.

**6.** The use of a compound as defined in claim 1, or a therapeutically acceptable salt thereof, for the preparation of a pharmaceutical composition for treating a herpes viral infection in a mammal.

**7.** The in vitro use of a compound as defined in claim 1, or a therapeutically acceptable salt thereof, for inhibiting the replication of herpes virus.

**8.** A compound as defined in claim 3 selected from the group consisting of {{(1α,2α,6α)-2,6-dimethyl-1-cyclohexanamino}carbonyl)-Tbg-$CH_2$-(R)-CH($CH_2$-C(O)$CMe_3$)C(O)-Asp(cyPn)-NH-(R)-CH(Et)$CMe_3$, {{(1α,2α,6α)-2,6-diethyl-1-cyclohexanamino}carbonyl}-Tbg-$CH_2$-(R)-CH($CH_2$C(O)$CMe_3$)C(O)-Asp(cyPn)-NH-(R)-CH(Et)$CMe_3$, and {{(1α,2α,4α,6α)-2,4,6-trimethyl-1-cyclohexanamino}carbonyl}-Tbg-$CH_2$-(R)-CH($CH_2$C(O)$CMe_3$)C(O)-Asp(cyPn)-NH-(R)-CH (Et)$CMe_3$ wherein Tbg represents the amino acid residue of (S)-2-amino-3, 3-dimethylbutanoic acid and Asp(cyPn) represents the amino acid residue of (S)-α-amino-1-carboxycyclopentaneacetic acid.

**9.** A pharmaceutical composition comprising an antiherpes virally effective amount of a compound as defined in claim 8, or a therapeutically acceptable salt thereof, and a pharmaceutically or veterinarily acceptable carrier.

**10.** A cosmetic composition comprising a compound as defined in claim 8, or a therapeutically acceptable salt thereof, and a physiologically acceptable carrier suitable for topical application.

**11.** The use of a compound as defined in claim 8, or a therapeutically acceptable salt thereof, for the preparation of a pharmaceutical composition for treating a herpes viral infection in a mammal.

**12.** The in vitro use of a compound as defined in claim 8, or a therapeutically acceptable salt thereof, for inhibiting the replication of herpes virus.

**13.** A pharmaceutical composition comprising a pharmaceutically or veterinarily acceptable carrier, and an effective

amount of the combination of an antiviral nucleoside analog or a therapeutically acceptable salt thereof, and a ribonucleotide reductase inhibiting compound as defined in claim 1, or a therapeutically acceptable salt thereof.

**14.** A pharmaceutical composition of claim 13 wherein the nucleoside analog is a compound of formula 19

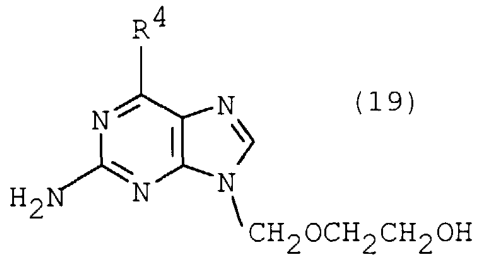

(19)

wherein $R^4$ is hydrogen, hydroxy or amino, or a therapeutically acceptable salt thereof.

**15.** A pharmaceutical composition of claim 13 wherein the antiviral nucleoside analog is selected from the group of penciclovir, famciclovir and valacyclovir.

**16.** The use of a combination of an antiviral nucleoside analog, or a therapeutically acceptable salt thereof, and a compound of formula 1 of claim 1, or a therapeutically acceptable salt thereof, for the preparation of a pharmaceutical composition for treating herpes viral infections in a mammal.

**17.** The use of claim 16 wherein the antiviral nucleoside analog is selected from the group of acyclovir, 6-deoxyacyclovir, 2,6-diamino-9-{(2-hydroxyethoxy)methyl}purine, penciclovir, famciclovir and valacyclovir.

**18.** The use of a compound as defined in claim 8 for the preparation of a pharmaceutical composition for treating herpes simplex virus type 1 or type 2 infections in a mammal.

**19.** A process for preparing a compound as defined in claim 1, or a therapeutically acceptable salt thereof, comprising:

(a) coupling a carboxylic acid derivative of formula 10

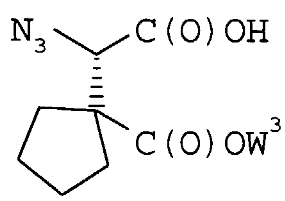

(10)

wherein $W^3$ is a carboxyl protective group with an amine of formula 11

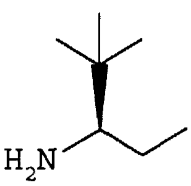

(11)

to obtain an α-azidoamide of formula 12

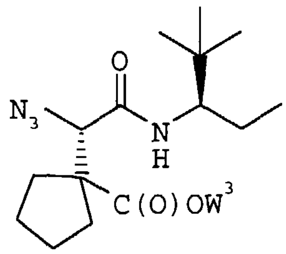

(12)

wherein $W^3$ is as defined in this claim,

(b) reducing the α-azidoamide of formula 12 to obtain a corresponding α-aminoamide of formula 13

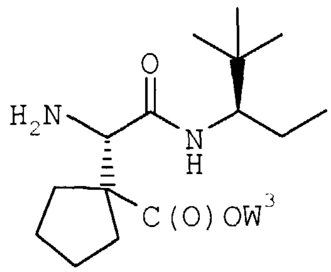

(13)

wherein $W^3$ is as defined in this claim,

(c) coupling the α-amidoamide of formula 13 with a carboxvlic acid derivative of formula 14

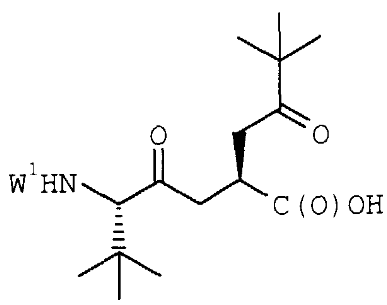

(14)

wherein $W^1$ is an amino protective group to obtain a diprotected intermediate of formula 15

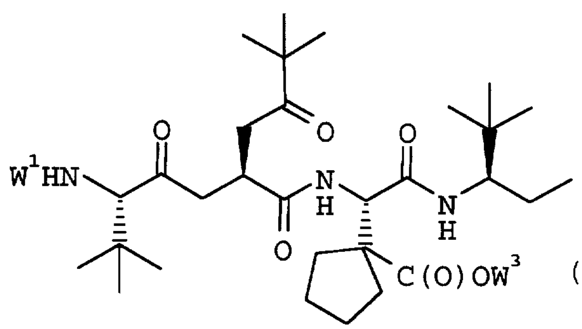

(15)

wherein $W^1$ and $W^3$ are as defined in this claim,

(d) selectively deprotecting the diprotected intermediate of formula 15 to obtain the free N-terminal derivative

of formula 16

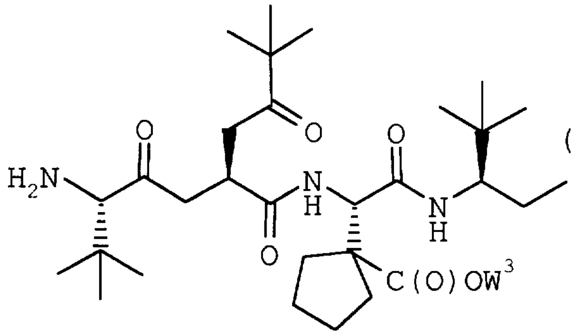

(16)

wherein $W^3$ is as defined in this claim,

(e) reacting the free N-terminal derivative of formula 16 with an isocyanatocyclohexane derivative of formula 17

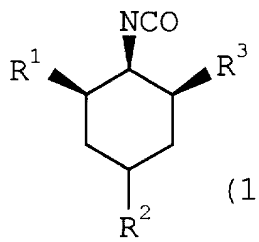

(17)

wherein $R^1$, $R^2$ and $R^3$ are as defined in claim 1 to obtain a ureido derivative of formula 18

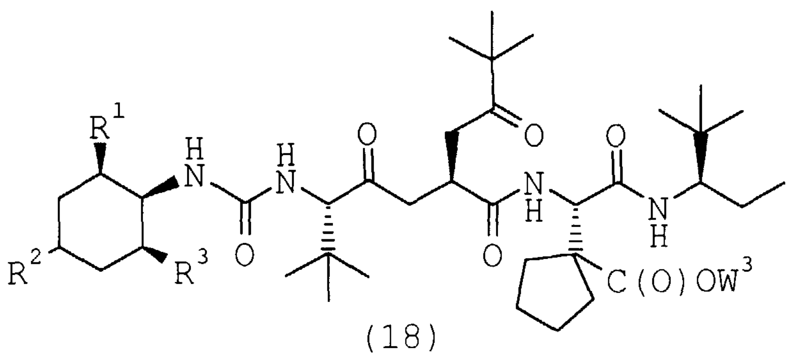

(18)

wherein $R^1$, $R^2$, $R^3$ and $W^3$ are as defined in this claim, and

(f) deprotecting the latter ureido derivative to obtain the corresponding compound of formula 1, and

(g) if desired transforming the compound of formula 1 into a therapeutically acceptable salt.

**20.** The process of claim 19 wherein $W^1$ is tert-butyloxycarbonyl and $W^3$ is benzyl.

**21.** An essentially pure enantiomorphic compound of the formula 2

$$W^1\text{-Tbg-}CH_2\text{-(R)-}CH(CH_2C(O)CMe_3)C(O)OW^2 \qquad (2)$$

wherein $W^1$ is an amino protective group, $W^2$ is a carboxyl protective group and Tbg represents the amino acid residue of (S)-2-amino-3,3-dimethylbutanoic acid.

**22.** A compound of formula 2 as defined in claim 21 wherein $W^1$ is tert-butyloxycarbonyl, phenylmethoxycarbonyl or 2,2,2-trichloro-ethoxycarbonyl, and $W^2$ is benzyl, (4-nitrophenyl)methyl, methyl or ethyl.

**23.** A process for preparing a compouna of formula 2 as defined in claim 21 comprising:

(a) reacting $W^1$-Tbg-(*E*)-CH=CHC(O)O$W^2$ wherein $W^1$, $W^2$ and Tbg are as defined in claim 21 with the sodium enolate of $CH_2=CHCH_2OC(O)CH_2C(O)CMe_3$ to obtain a Michael adduct of formula

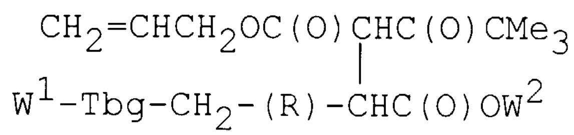

and (b) reacting the Michael adduct with tetrakistriphenylphosphine palladium(O) in the presence of a secondary amine to obtain the compound of formula 2.

**Patentansprüche**

**1.** Verbindung der Formel 1

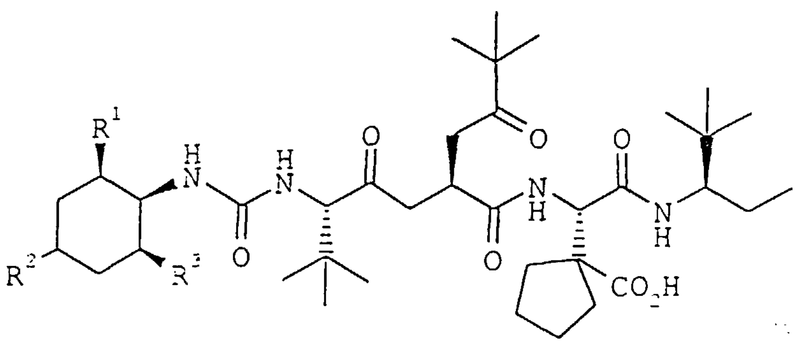

(1)

worin $R^1$ (1-3C)Alkyl, $R^2$ Wasserstoff oder (1-3C)Alkyl, und $R^3$ (1-3C)Alkyl bedeutet; oder ein therapeutisch annehmbares Salz davon.

**2.** Verbindung nach Anspruch 1, wobei $R^1$ und $R^3$ beide Methyl oder beide Ethyl bedeuten und $R^2$ Wasserstoff, Methyl oder Ethyl bedeutet; oder ein therapeutisch annehmbares Salz davon.

**3.** Verbindung nach Anspruch 2, wobei $R^1$ und $R^3$ beide Methyl bedeuten und $R^2$ Wasserstoff oder Methyl in Position *cis* zu $R^1$ und $R^3$ bedeutet; oder wobei $R^1$ und $R^3$ beide Ethyl bedeuten und $R^2$ Wasserstoff bedeutet; oder ein therapeutisch annehmbares-Salz davon.

**4.** Arzneimittelzusammensetzung, enthaltend eine gegen Herpes antiviral wirksame Menge einer wie in Anspruch 1 definierten Verbindung oder eines therapeutisch annehmbaren Salzes davon, sowie einen pharmazeutisch oder veterinärmedizinisch annehmbaren Träger.

**5.** Kosmetische Zusammensetzung, enthaltend eine wie in Anspruch 1 definierte Verbindung oder ein therapeutisch annehmbares Salz davon, sowie einen physiologisch annehmbaren, zur topischen Auftragung geeigneten Träger.

**6.** Verwendung einer wie in Anspruch 1 definierten Verbindung oder eines therapeutisch annehmbaren Salzes davon

zur Herstellung einer Arzneimittelzusammensetzung zur Behandlung einer Herpes-viralen Infektion bei einem Säuger.

**7.** In-vitro-Verwendung einer wie in Anspruch 1 definierten Verbindung oder eines therapeutisch annehmbaren Salzes davon zur Hemmung der Replikation des Herpes-Virus.

**8.** Verbindung, wie im Anspruch 3 definiert, ausgewählt aus der Gruppe, die von {{(1α,2α,6α)-2,6-Dimethyl-1-cyclohexanamino)carbonyl)-Tbg-$CH_2$-(R)-CH($CH_2$C(O)$CMe_3$)C(O)-Asp(cyPn)-NH-(R)-CH(Et)$CMe_3$, {{(1α,2α,6α)-2,6-Diethyl-1-cyclohexanamino}carbonyl)-Tbg-$CH_2$-(R)-CH($CH_2$C(O)$CMe_3$)C(O)-Asp(cyPn)-NH-(R)-CH(Et)$CMe_3$ und {{(1α,2α,4α,6α)-2,4,6-Trimethyl-1-cyclohexanamino)carbonyl)-Tbg-$CH_2$-(R)-CH($CH_2$C(O)-$CMe_3$)C(O)-Asp(cyPn)-NH-(R)-CH(Et)$CMe_3$ gebildet ist, wobei Tbg den Aminosäurerest der (S)-2-Amino-3,3-dimethylbuttersäure und Asp (cyPn) den Aminosäurerest der (S)-α-Amino-1-carboxycyclopentanessigsäure bedeutet.

**9.** Arzneimittelzusammensetzung, enthaltend eine gegen Herpes antiviral wirksame Menge einer wie in Anspruch 8 definierten Verbindung oder eines therapeutisch annehmbaren Salzes davon, sowie einen pharmazeutisch oder veterinärmedizinisch annehmbaren Träger.

**10.** Kosmetische Zusammensetzung, enthaltend eine wie in Anspruch 8 definierte Verbindung oder ein therapeutisch annehmbares Salz davon, sowie einen physiologisch annehmbaren, zur topischen Auftragung geeigneten Träger.

**11.** Verwendung einer wie in Anspruch 8 definierten Verbindung oder eines therapeutisch annehmbaren Salzes davon zur Herstellung einer Arzneimittelzusammensetzung zur Behandlung einer Herpes-viralen Infektion bei einem Säuger.

**12.** In-vitro-Verwendung einer wie in Anspruch 8 definierten Verbindung oder eines therapeutisch annehmbaren Salzes davon zur Hemmung der Replikation des Herpes-Virus.

**13.** Arzneimittelzusammensetzung, enthaltend einen pharmazeutisch oder veterinärmedizinisch annehmbaren Träger und eine wirksame Menge einer Kombination eines antiviralen Nucleosidanalogons oder eines therapeutisch annehmbaren Salzes davon und einer wie in Anspruch 1 definierten, die Ribonucleotid Reductase hemmende Verbindung oder eines therapeutisch annehmbaren Salzes davon.

**14.** Arzneimittelzusammensetzung nach Anspruch 13, wobei das Nucleosidanalogon eine Verbindung der Formel 19

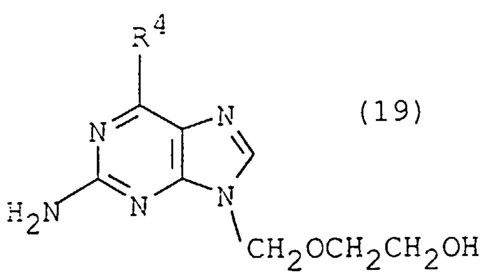

(19)

ist, worin $R^4$ Wasserstoff, Hydroxy oder Amino bedeutet, oder ein therapeutisch annehmbares Salz davon ist.

**15.** Arzneimittelzusammensetzung nach Anspruch 13, wobei das antivirale Nucleosidanalogon aus der von Penciclovir, Famciclovir und Valacyclovir gebildeten Gruppe ausgewählt ist.

**16.** Verwendung einer Kombination eines antiviralen Nucleosidanalogons oder eines therapeutisch annehmbaren Salzes davon und einer Verbindung der Formel 1 nach Anspruch 1 oder eines therapeutisch annehmbaren Salzes davon zur Herstellung einer Arzneimittelzusammensetzung zur Behandlung einer Herpes-viralen Infektion bei einem Säuger.

**17.** Verwendung nach Anspruch 16, wobei das antivirale Nucleosidanalogon aus der von Acyclovir, 6-Deoxyacyclovir,

2,6-Diamino-9-{(2-hydroxyethoxy)methyl}purin, Penciclovir, Famciclovir und Valacyclovir gebildeten Gruppe ausgewählt ist.

**18.** Verwendung einer wie in Anspruch 8 definierten Verbindung zur Herstellung einer Arzneimittelzusammensetzung zur Behandlung von Infektionen durch das Herpesvirus des Typus 1 oder 2 bei einem Säuger.

**19.** Verfahren zur Herstellung einer wie in Anspruch 1 definierten Verbindung oder eines therapeutisch annehmbaren Salzes davon, umfassend:

(a) Kopplung eines Derivats einer Carbonsäure der Formel 10

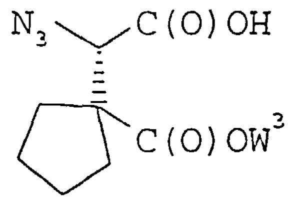

(10)

worin $W^3$ eine Carboxylschutzgruppe bedeutet, mit einem Amin der Formel 11

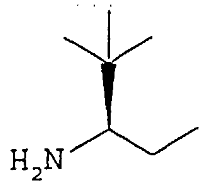

(11)

zum Erhalt eines α-Azidoamids der Formel 12

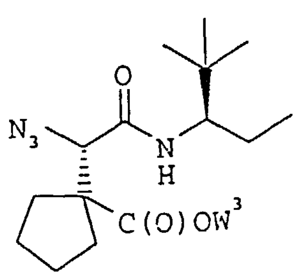

(12)

worin $W^3$ wie im vorliegenden Anspruch definiert ist,

(b) Reduktion des α-Azidoamids der Formel 12 zum Erhalt eines entsprechenden α-Aminoamids der Formel 13

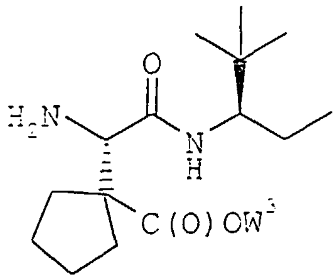

(13)

worin $W^3$ wie im vorliegenden Anspruch definiert ist,

(c) Kopplung des α-Aminoamids der Formel 13 mit einem Derivat einer Carbonsäure der Formel 14

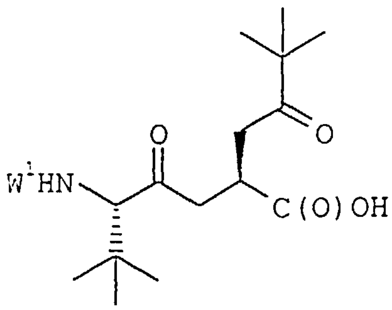

(14)

wobei $W^1$ eine Aminoschutzgruppe bedeutet, zum Erhalt einer doppelt geschützten Zwischenverbindung der Formel 15

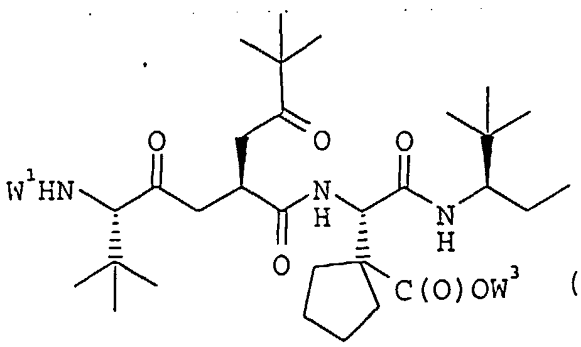

(15)

worin $W^1$ und $W^3$ wie im vorliegenden Anspruch definiert sind,

(d) Selektive Schutzentfernung von der doppelt geschützten Zwischenverbindung der Formel 15 zum Erhalt des Derivats mit freiem endständigen N der Formel 16

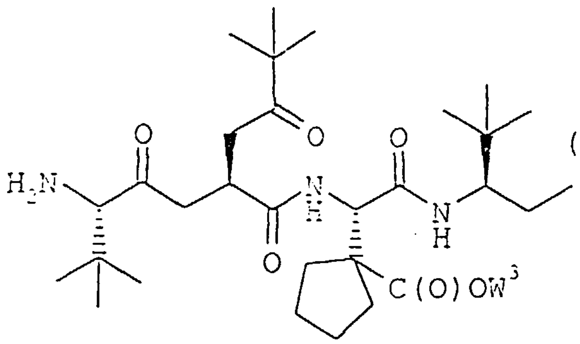

(16)

worin $W^3$ wie im vorliegenden Anspruch definiert ist,

(e) Umsetzung des Derivats mit freiem endständigen N der Formel 16 mit einem Isocyanatocyclohexan-Derivat der Formel 17

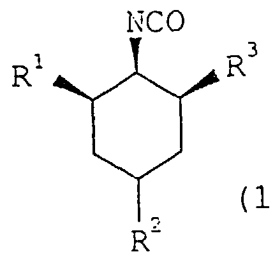

(17)

worin $R^1$, $R^2$ und $R^3$ wie im Anspruch 1 definiert sind, zum Erhalt eines Ureido-Derivats der Formel 18

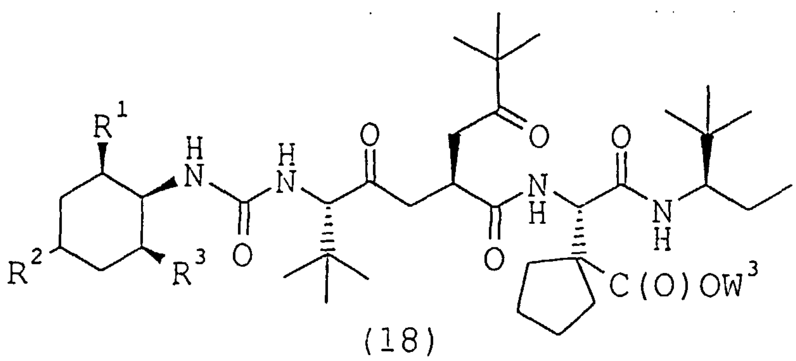

(18)

worin $R^1$, $R^2$, $R^3$ und $W^3$ wie im vorliegenden Anspruch definiert sind, und

(f) Schutzentfernung vom letztgenannten Ureido-Derivat zum Erhalt der entsprechenden Verbindung der Formel 1, und

(g) falls erwünscht, Umsetzung der Verbindung der Formel 1 zu einem therapeutisch annehmbaren Salz.

**20.** Verfahren nach Anspruch 19, wobei $W^1$ tert-Butyloxycarbonyl und $W^3$ Benzyl bedeutet.

**21.** Im wesentlichen reine enanthiomorphe Verbindung der Formel 2

$$W^1\text{-Tbg-}CH_2\text{-(R)-}CH(CH_2C(O)CMe_3)C(O)OW^2 \quad (2)$$

worin $W^1$ eine Aminoschutzgruppe, $W^2$ eine Carboxylschutzgruppe und Tbg den Aminosäurerest der (S)-2-Amino-3,3-dimethylbuttersäure bedeutet.

**22.** Verbindung der Formel 2, wie im Anspruch 21 definiert, wobei $W^1$ *tert*-Butyloxycarbonyl, Phenylmethoxycarbonyl oder 2,2,2-Trichlorethoxycarbonyl und $W^2$ Benzyl, (4-Nitrophenyl)methyl, Methyl oder Ethyl bedeutet.

**23.** Verfahren zur Herstellung einer wie in Anspruch 21 definierten Verbindung der Formel 2, umfassend:

(a) Umsetzung von $W^1$-Tbg-*(*E)-CH=CHC(O)O$W^2$, wobei $W^1$, $W^2$ und Tbg wie im Anspruch 21 definiert sind, mit dem Natriumenolat von $CH_2=CHCH_2OC(O)CH_2C(O)CMe_3$ zum Erhalt eines Michael-Addukts der Formel

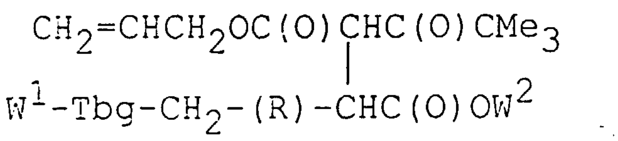

und (b) Umsetzung des Michael-Addukts mit Tetrakistriphenylphosphinpalladium(0) in Gegenwart eines sekundären Amins zum Erhalt der Verbindung der Formel 2.

## Revendications

**1.** Composé de formule 1

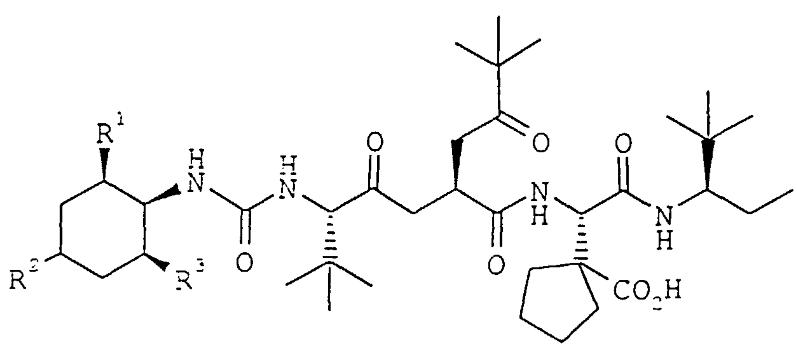

(1)

dans laquelle $R^1$ représente un (1-3C)alkyle, $R^2$ représente un hydrogène ou (1-3C)alkyle, et $R^3$ représente un (1-3C)alkyle; ou bien un sel thérapeutiquement acceptable dudit composé.

**2.** Composé selon la revendication 1 avec $R^1$ et $R^3$ représentant tous les deux un méthyle ou tous les deux un éthyle, et avec $R^2$ représentant un hydrogène, méthyle ou éthyle; ou bien un sel thérapeutiquement acceptable dudit composé.

**3.** Composé selon la revendication 2 avec $R^1$ et $R^3$ représentant tous les deux un méthyle et $R^2$ représentant un hydrogène ou un méthyle en position *cis* par rapport à $R^1$ et $R^3$; ou bien avec $R^1$ et $R^3$ représentant tous les deux un éthyle et $R^2$ représentant un hydrogène; ou bien un sel thérapeutiquement acceptable dudit composé.

**4.** Composition pharmaceutique comportant une quantité antivirale efficace anti-herpès d'un composé tel que défini dans la revendication 1 ou bien d'un sel thérapeutiquement acceptable dudit composé, ainsi qu'un support acceptable pour l'usage pharmaceutique ou vétérinaire.

**5.** Composition cosmétique comportant un composé tel que défini dans la revendication 1 ou bien un sel thérapeutiquement acceptable dudit composé, ainsi qu'un support physiologiquement acceptable approprié à l'application topique.

**6.** Utilisation d'un composé tel que défini dans la revendication 1 ou bien d'un sel thérapeutiquement acceptable dudit composé pour la préparation d'une composition pharmaceutique destinée à traiter une infection virale par l'herpès chez un mammifère.

**7.** Utilisation in vitro d'un composé tel que défini dans la revendication 1 ou bien d'un sel thérapeutiquement acceptable dudit composé pour inhiber la réplication du virus de l'herpès.

**8.** Composé tel que défini dans la revendication 3, choisi parmi le groupe constitué de {{(1α,2α,6α)-2,6-diméthyl-1-cyclohexaneamino)carbonyl)-Tbg-$CH_2$-(R)-CH($CH_2$C(O)$CMe_3$)C(O)-Asp(cyPn)-NH-(R)-CH(Et)$CMe_3$, {{(1α,2α,6α)-2,6-diéthyl-1-cyclohexaneamino)carbonyl)-Tbg-$CH_2$-(R)-CH($CH_2$C(O)$CMe_3$)C(O)-Asp(cyPn)-NH-(R)-CH(Et)$CMe_3$ et {{(1α,2α,4α,6α)-2,4,6-triméthyl-1-cyclohexaneamino)carbonyl)-Tbg-$CH_2$-(R)-CH($CH_2$C(O)-$CMe_3$)C(O)-Asp(cyPn)-NH-(R)-CH(Et)$CMe_3$, avec Tbg représentant le résidu acide aminé de l'acide (S)-2-amino-3,3-diméthylbutyrique et Asp(cyPn) représentant le résidu acide aminé de l'acide (S)-α-amino-1-carboxycyclopentaneacétique.

**9.** Composition pharmaceutique comportant une quantité antivirale efficace anti-herpès d'un composé tel que défini dans la revendication 8 ou bien d'un sel thérapeutiquement acceptable dudit composé, ainsi qu'un support acceptable pour l'usage pharmaceutique ou vétérinaire.

**10.** Composition cosmétique comportant un composé tel que défini dans la revendication 8 ou bien un sel thérapeutiquement acceptable dudit composé, ainsi qu'un support physiologiquement acceptable approprié à l'application topique.

**11.** Utilisation d'un composé tel que défini dans la revendication 8 ou bien d'un sel thérapeutiquement acceptable dudit composé pour la préparation d'une composition pharmaceutique destinée à traiter une infection virale par l'herpès chez un mammifère.

**12.** Utilisation in vitro d'un composé tel que défini dans la revendication 8 ou bien d'un sel thérapeutiquement acceptable dudit composé pour inhiber la réplication du virus de l'herpès.

**13.** Composition pharmaceutique comportant un support acceptable pour l'usage pharmaceutique ou vétérinaire et une quantité efficace d'une combinaison d'un composé antiviral analogue de nucléoside ou bien d'un sel thérapeutiquement acceptable dudit composé et d'un composé inhibiteur de la ribonucléotide réductase tel que défini dans la revendication 1 ou bien d'un sel thérapeutiquement acceptable dudit composé.

**14.** Composition pharmaceutique selon la revendication 13 dans laquelle le composé analogue de nucléoside est un composé de formule 19

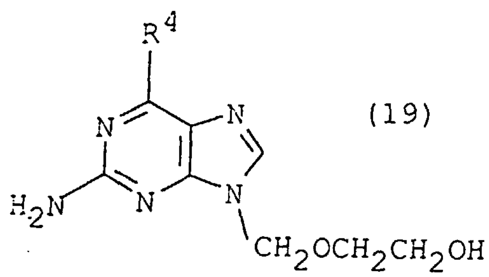

(19)

dans laquelle $R^4$ représente un hydrogène, hydroxy ou amino; ou bien un sel thérapeutiquement acceptable dudit composé.

**15.** Composition pharmaceutique selon la revendication 13 dans laquelle le composé analogue de nucléoside est choisi dans le groupe composé de penciclovir, famciclovir et valacyclovir.

**16.** Utilisation d'une combinaison d'un composé antiviral analogue de nucléoside ou bien d'un sel thérapeutiquement acceptable dudit composé et d'un composé selon la formule 1 de la revendication 1 ou bien d'un sel thérapeutiquement acceptable dudit composé pour la préparation d'une composition pharmaceutique destinée à traiter des infections virales par l'herpès chez un mammifère.

**17.** Utilisation selon la revendication 16 dans laquelle le composé antiviral analogue de nucléoside est choisi dans le groupe composé d'acyclovir, 6-désoxyacyclovir, 2,6-diamino-9-{(2-hydroxyéthoxy)méthyl}purine, penciclovir, famciclovir et valacyclovir.

**18.** Utilisation d'un composé tel que défini dans la revendication 8 pour la préparation d'une composition pharmaceutique destinée à traiter des infections par le virus herpès simplex du type 1 ou 2 chez un mammifère.

**19.** Procédé de préparation d'un composé tel que défini dans la revendication 1 ou bien d'un sel thérapeutiquement acceptable dudit composé, comportant:

(a) le couplage d'un dérivé d'acide carboxylique de formule 10

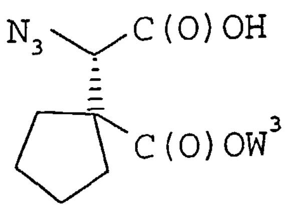

(10)

dans laquelle $W^3$ représente un groupe protecteur de carboxyle avec une amine de formule 11

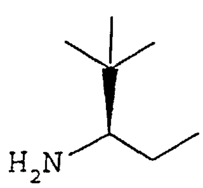

(11)

pour obtenir une α-azidoamide de formule 12

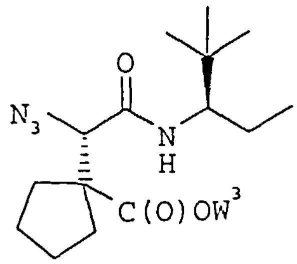

(12)

dans laquelle $W^3$ est tel que défini dans la présente revendication,

(b) la réduction de l'α-azidoamide de formule 12 pour obtenir une α-aminoamide correspondante de formule 13

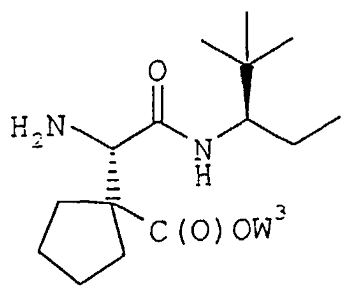

(13)

dans laquelle $W^3$ est tel que défini dans la présente revendication,

(c) le couplage de l'α-aminoamide de formule 13 avec un dérivé d'acide carboxylique de formule 14

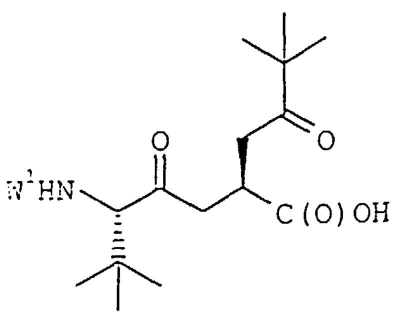

(14)

dans laquelle $W^1$ représente un groupe protecteur d'amine pour obtenir un composé intermédiaire doublement protégé de formule 15

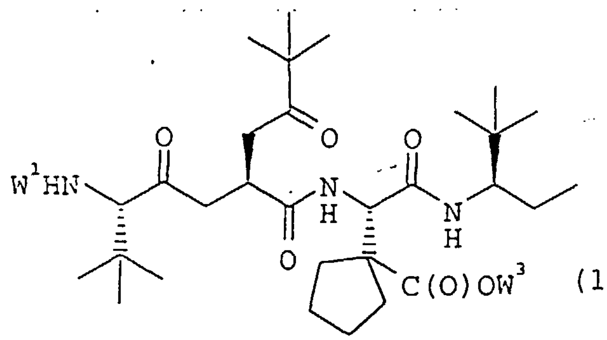

(15)

dans laquelle $W^1$ et $W^3$ sont tels que définis dans la présente revendication,

(d) l'élimination sélective de la protection du composé intermédiaire doublement protégé de formule 15 pour obtenir le dérivé à N terminal libre de formule 16

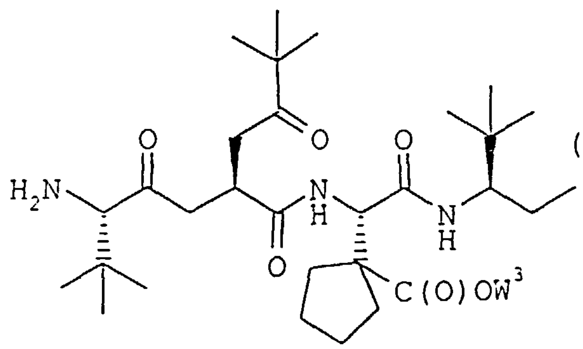

(16)

dans laquelle $W^3$ est tel que défini dans la présente revendication,

(e) la mise en réaction du dérivé à N terminal libre de formule 16 avec un dérivé d'isocyanatocyclohexane de formule 17

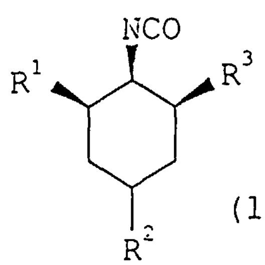

(17)

dans laquelle $R^1$, $R^2$ et $R^3$ sont tels que définis dans la revendication 1 pour obtenir un dérivé uréido de formule 18

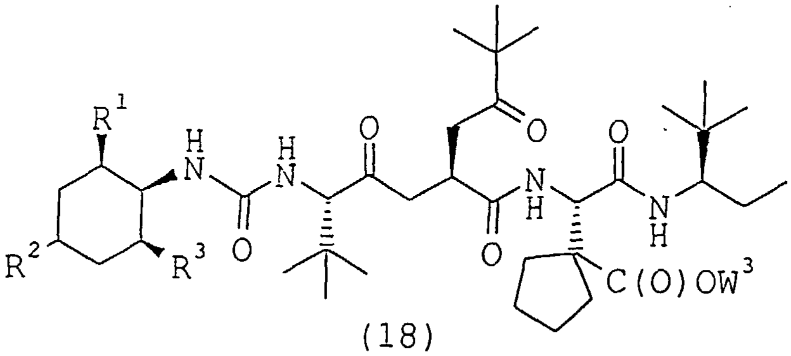

(18)

dans laquelle $R^1$, $R^2$, $R^3$ et $W^3$ sont tels que définis dans la présente revendication, et

(f) l'élimination de la protection de ce dernier dérivé uréido pour obtenir le composé correspondant de formule 1, et

(g) si on le désire, la transformation du composé de formule 1 en un sel thérapeutiquement acceptable.

**20.** Procédé selon la revendication 19 avec $W^1$ représentant un *tert*-butyloxycarbonyle et $W^3$ représentant un benzyle.

**21.** Composé énanthiomorphe essentiellement pur de formule 2

$$W^1\text{-Tbg-}CH_2\text{-(R)-}CH(CH_2C(O)CMe_3)C(O)OW^2 \qquad (2)$$

dans laquelle $W^1$ représente un groupe protecteur d'amine, $W^2$ représente un groupe protecteur de carboxyle et Tbg représente le résidu acide aminé de l'acide (S)-2-amino-3,3-diméthylbutyrique.

**22.** Composé de formule 2 tel que défini dans la revendication 21 avec $W^1$ représentant un *tert*-butyloxycarbonyle, phénylméthoxycarbonyle ou 2,2,2-trichloroéthoxycarbonyle et $W^2$ représentant un benzyle, (4-nitrophényl)méthyle, méthyle ou éthyle.

**23.** Procédé de préparation d'un composé de formule 2 tel que défini dans la revendication 21, comportant:

(a) la mise en réaction du composé $W^1$-Tbg-(E)-CH=CHC(O)O$W^2$ avec $W^1$, $W^2$ et Tbg tels que définis dans la revendication 21 avec l'énolate de sodium du composé $CH_2$=$CHCH_2OC(O)CH_2C(O)CMe_3$ pour obtenir un produit d'addition de Michael de formule

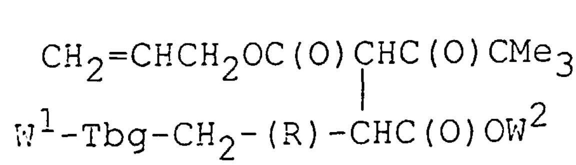

et (b) la mise en réaction du produit d'addition de Michael avec le tétrakistriphénylphosphinepalladium(0) en présence d'une amine secondaire pour obtenir le composé de formule 2.

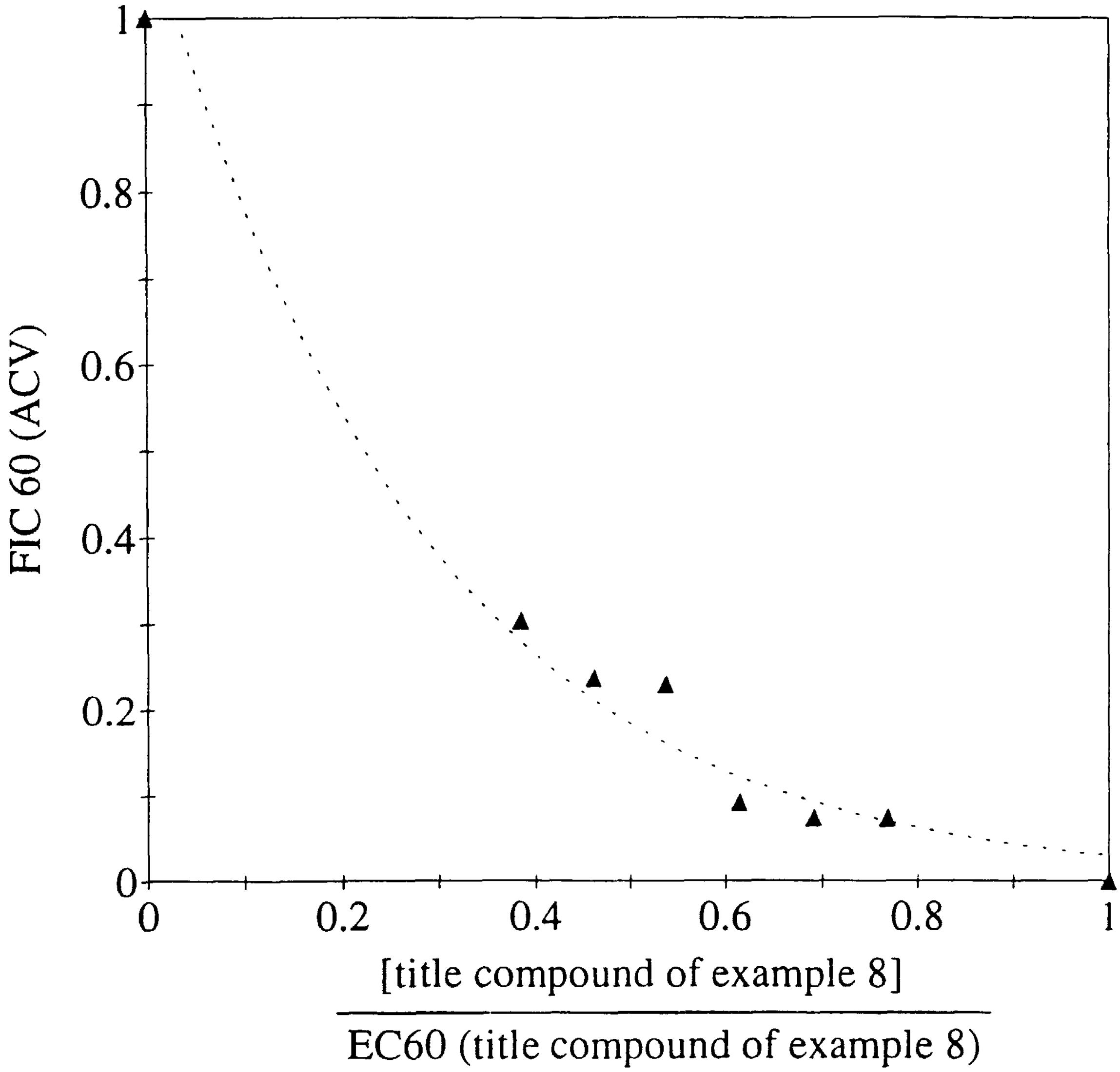
1
0.8
0.6
0.4
0.2
0
FIC 60 (ACV)
0
0.2
0.4
0.6
0.8
1
[title compound of example 8]
EC60 (title compound of example 8)

*Figure 1*

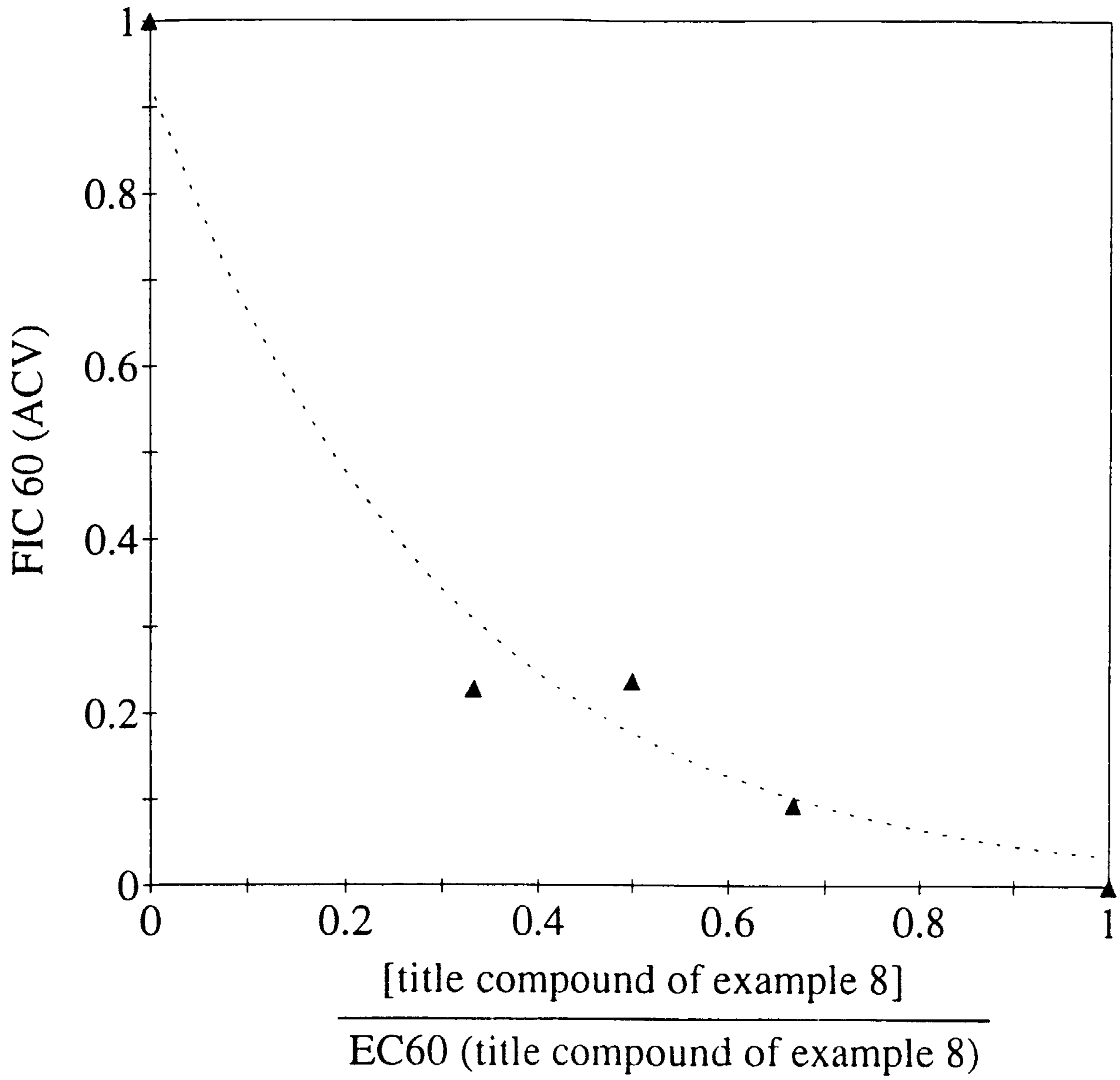
1
0.8
0.6
0.4
0.2
0
FIC 60 (ACV)
0
0.2
0.4
0.6
0.8
1
[title compound of example 8]
EC60 (title compound of example 8)

*Figure 2*